# EUROPEAN PATENT APPLICATION

(11) **EP 4 066 769 A1**
(43) Date of publication of application: **05.10.2022**
(21) Application number: 21305417.4
(22) Date of filing: 31.03.2021
(51) Int. Cl.: A61B 34/00, A61B 34/30, A61B 90/50, A61B 90/57, A61B 90/90, A61B 90/98, A61B 46/10

(54) **COUPLERS AND MODES OF OPERATION FOR ROBOTIC SYSTEMS**

(71) Applicant: Moon Surgical, 75009 Paris (FR)
(72) Inventor: Noonan, David Paul, 75009 Paris (FR); Alvarez, Jeffery Byron, 75009 Paris (FR); Basafa, Ehsan, 75009 Paris (FR); Tanner, Neal, 75009 Paris (FR); Linard, Nicolas, 75009 Paris (FR)
(74) Representative: Brevalex

(57) **Abstract**

**ABSTRACT OF THE DISCLOSURE**

Devices for coupling an instrument to an arm of a surgical robot include a coupler body configured to couple with an instrument for use in a surgical operation and a coupler interface configured to selectively receive the coupler body and configured to be coupled with an end portion of a robotic arm. The device can be configured to permit the instrument to rotate about a longitudinal axis of the instrument relative to the device. The device can be configured to at least inhibit longitudinal movement of the instrument relative to the device. Robotic systems include one or more arms of the system controllable according to a plurality of selectable and different operational modes such as a passive assistant mode, a co-manipulation assistant mode, a robotic assistant mode, and/or a haptic mode.

## Description

### FIELD

This disclosure relates generally to robotic systems, and in particular, to co-manipulation surgical robotic systems.

### SUMMARY

In some embodiments, a device for selectively coupling an instrument to an arm of a surgical robot can include a body configured to couple with an instrument for use in a surgical operation (for example and without limitation, a laparoscopic surgical instrument or an endoscope) and an interface configured to selectively receive the body and configured to be coupled with an end portion of a robotic arm. The device can be configured to permit the instrument to rotate about a longitudinal axis of the instrument relative to the device and to inhibit longitudinal movement of the instrument relative to the coupler.

In some embodiments, the body can be configured to clamp around a portion of an outside surface of the instrument. The body can have a recess therein configured to receive the instrument. The recess can extend all the way through the body. The body can include an interface (e.g., a gripping interface) configured to grip an outside surface of the instrument.

The body can include a first portion coupled with a second portion with a hinge, wherein the first portion can rotate about the hinge relative to the second portion so as to selectively clamp the instrument in a recess formed in the body. In some embodiments, the body can be configured to clamp around a portion of an outside surface of the instrument and to prevent a rotational movement of the instrument relative to the body under normal operating conditions. In some embodiments, the interface can include a recess configured to removably receive the body therein. The body can be selectively secured in the recess of the interface. In some embodiments, the recess of the interface can be configured to inhibit longitudinal movement of the body relative to the interface and to permit rotational movement of the body relative to the interface. The device of some embodiments can move between a first state in which the instrument is removable from the device and a second state in which the instrument is coupled with or nonremovable from the device. In some embodiments, the device can be biased toward the second state. In any embodiments disclosed herein, body can have one or more projections extending away from a surface of the body and the interface can have one or more depressions configured to receive one or more of the projections to align the body with the interface.

Some embodiments of a kit disclosed herein may include any one of the couplers described herein and a surgical instrument. Some embodiments of a kit disclosed herein may include any one of the couplers described herein and a robotic arm. Some embodiments of a method disclosed herein may include operating any one of the couplers described herein. Some embodiments of a device, system and/or method may be as illustrated and/or described herein.

In some embodiments, a method of operating a co-manipulation robotic surgical device can include coupling an instrument to an end portion of an arm of the robotic surgical device and selecting an operational mode of the surgical device from a list comprising at least two of the following operational modes: co-manipulation assistant mode, passive assistant mode, robotic assistant mode, and a haptic mode. The method can further include performing a surgical operation using the instrument coupled to the distal end of the arm of the robotic surgical device.

Some embodiments of a co-manipulation robotic surgical device for manipulating a surgical instrument can include a base portion, a first arm coupled with the base portion, a motor coupled with the first arm and configured to rotate the first arm relative to the base portion, an instrument coupled with an end portion of the first arm, and a controller configured to control the first arm according to at least two of the following operational modes: passive assistant mode, co-manipulation assistant mode, robotic assistant mode, and haptic mode.

In some embodiments of the method or system, when the robotic surgical device is in the passive assistant mode, the arm is static. In some embodiments, when the robotic surgical device is in the co-manipulation assistant mode, the robotic surgical device may be configured such that one or more arms (e.g., the first arm) of the robotic surgical device may be freely moved by an operator and may be at least partially simultaneously moved by the surgical device (e.g., based on algorithmic computations). In some embodiments, when the robotic surgical device is in the co-manipulation assistant mode, one or more arms (e.g., the first arm) of the robotic surgical device is freely movable by an operator while the motor at least partially simultaneously moves the first arm to improve a position and/or orientation of the instrument coupled with the end portion of the first arm and/or to compensate at least for a force of gravity on the first arm and the instrument that is coupled with the end portion of the first arm.

In some embodiments, when the robotic surgical device is in the robotic assistant mode, the robotic surgical device can be configured such that one or more arms (e.g., the first arm) of the robotic surgical device may move to reposition the instrument coupled with the distal end of the arm of the surgical device (e.g., based on algorithmic computations). In some embodiments, when the robotic surgical device is in the haptic mode, the robotic surgical device may be configured such that one or more arms (e.g., the first arm) of the robotic surgical device may be simultaneously freely movable by an operator and moved by a motor of the robotic surgical device (e.g., based on algorithmic computations). In some embodiments, when the robotic surgical device is in the haptic mode, the first arm can be movable by an operator while the motor compensates at least for a force of gravity on the first arm and/or the instrument that is coupled with the end portion of the first arm and at least guides the instrument along a predefined trajectory, prevents unwanted movements of the first arm and/or the instrument coupled with the end portion of the first arm, prevents a movement of the first arm outside of a particular space, and/or prevents a movement of the first arm into a particular space.

In some embodiments, the robotic surgical device can be configured to automatically identify a particular instrument that is coupled with the end portion of the first arm. For example and without limitation, in some embodiments, the co-manipulation robotic surgical device can be configured to automatically identify the particular instrument that is coupled with the end portion of the first arm using an RFID transmitter chip, a barcode, a near field communication device, a Bluetooth transmitter, and/or a weight of the instrument that is coupled with the end portion of the first arm. Some embodiments of the robotic surgical device can be configured to automatically change to a predetermined one of the operational modes when a particular instrument is coupled with the end portion of the first arm, without any additional input from an operator. In some embodiments, the co-manipulation robotic surgical device can be configured to change to the passive assistant mode when a particular instrument (e.g., an endoscope) is coupled with the end portion of the first arm, without any additional input from an operator, or to change to the haptic mode when a particular instrument is coupled with the end portion of the first arm, without any additional input from an operator, or to change to the co-manipulation assistant mode when a particular instrument is coupled with the end portion of the first arm, without any additional input from an operator.

The method may include selecting an operational mode of the surgical device from the list comprising at least three of the following operational modes: co-manipulation assistant mode, passive assistant mode, robotic assistant mode, and haptic mode.

The method may include selecting an operational mode of the surgical device from the list comprising each of the following operational modes: co-manipulation assistant mode, passive assistant mode, robotic assistant mode, and haptic mode.

### BRIEF DESCRIPTION OF THE DRAWINGS

Figure 1A illustrates an embodiment of a robotic system.
Figures 1B-1K illustrate another example embodiment of a robotic system.
Figures 2A-2D illustrate an example embodiment of a coupler.
Figures 3A-3D illustrate another example embodiment of a coupler.
Figures 4A-4B illustrate another example embodiment of a coupler.
Figures 5A-5B illustrate another example embodiment of a coupler.
Figures 6A-6C illustrate another example embodiment of a coupler.
Figures 7A-7B illustrate another example embodiment of a coupler.
Figures 7C-7D illustrate another example embodiment of a coupler.
Figures 7E-7F illustrate another example embodiment of a coupler.
Figures 8A-8B illustrate another example embodiment of a coupler.
Figures 9A-9B illustrate another example embodiment of a coupler.
Figures 10A-10B illustrate another example embodiment of a coupler.
Figures 11-14 illustrate additional example embodiments of couplers.
Figure 15 illustrates an example embodiment of modes of operation for a robotic system.

### DETAILED DESCRIPTION

Embodiments of the robotic systems disclosed herein can be configured for co-manipulation wherein, in some embodiments, the robotic system can assist the operator (typically a surgeon, but can be a surgical assistant or other) by robotically moving one or more arms of the robotic system and/or one or more instruments coupled with the arm(s) of the robotic system in response to force inputs provided by the operator. In some nonlimiting examples of the co-manipulation surgical robotic system 100 (also referred to as a robot or a robotic system) disclosed herein, the robotic system 100 can be configured to assist in the manipulation of an instrument, such as a surgical instrument, camera, or any other instrument, in a surgical operation. Some examples of the robotic system 100 disclosed herein can be particularly useful in laparoscopic surgical procedures and/or other surgical procedures that utilize long and thin instruments that can be inserted (typically, via cannulas) into the body of a patient so as to allow surgical intervention; none of the embodiments of the robotic system 100 disclosed herein are so limited, but indeed can be used for any desired or suitable surgical operation. In some embodiments, the robotic system 100 can be used in conjunction or cooperation with video monitoring provided by one or more cameras and/or one or more endoscopes so that an operator of the robotic system 100 can see and monitor the use of the instrument coupled with an arm of the robotic system 100.

For example and without limitation, some embodiments the robotic system 100 can be configured so that it is not controlled remotely, yet the robotic system 100 supplies all or substantially all of the force to move or manipulate a surgical instrument. Some embodiments of the robotic system 100 can be configured to be controlled or manipulated remotely or, or in some operations of the robotic system 100 can be controlled and manipulated remotely - for example and without limitation, via joystick or other suitable remote control device, computer vision algorithm, force measuring algorithm, and/or by other means.

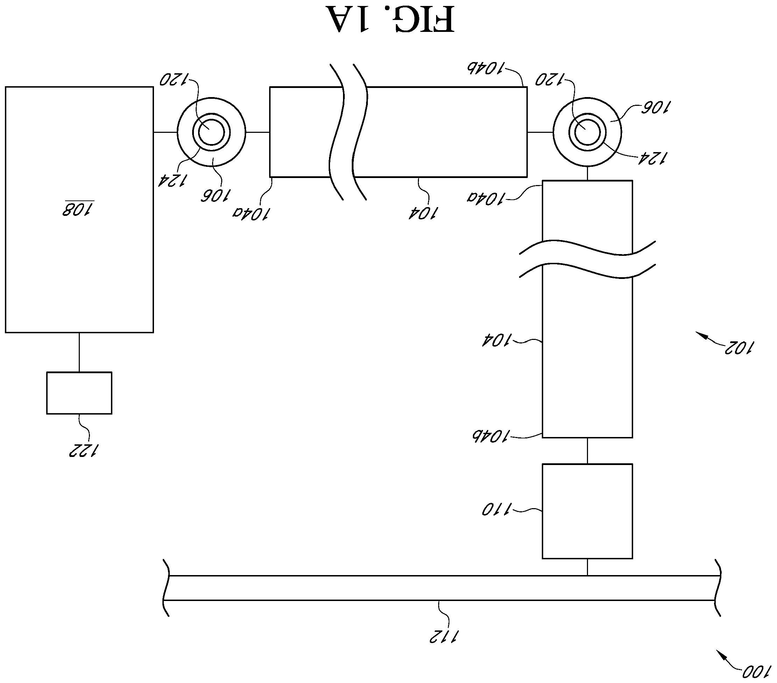

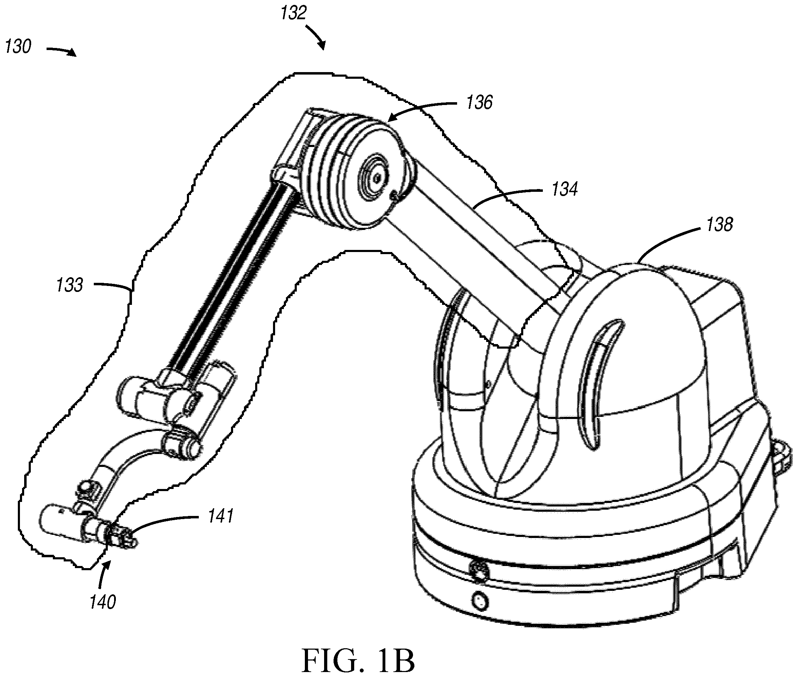

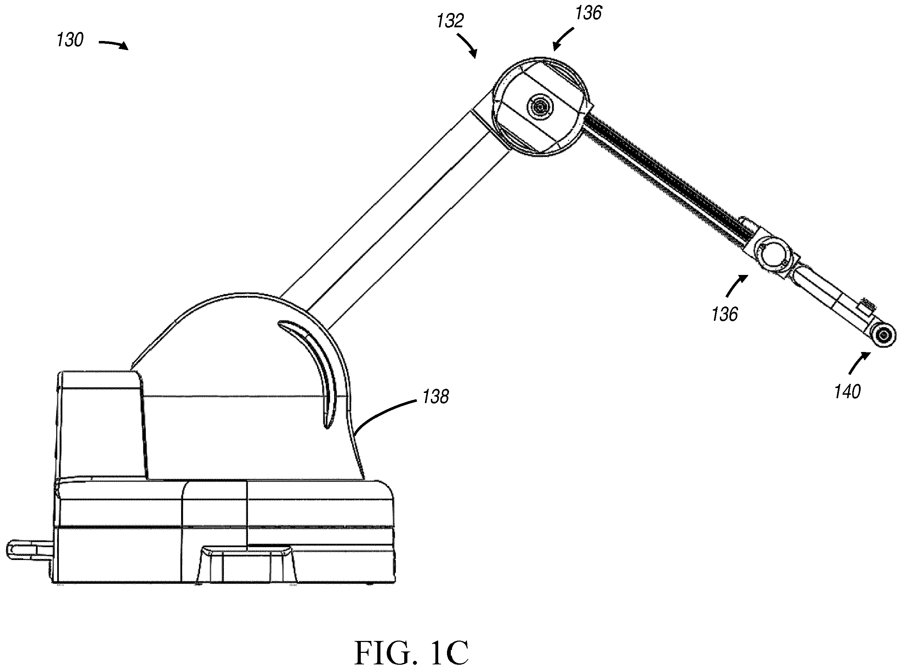

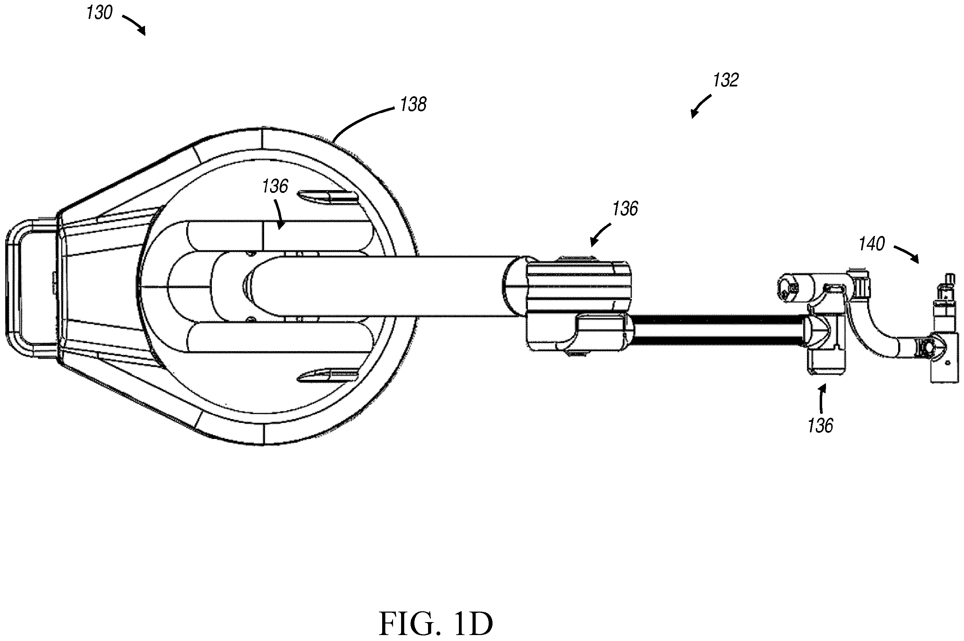

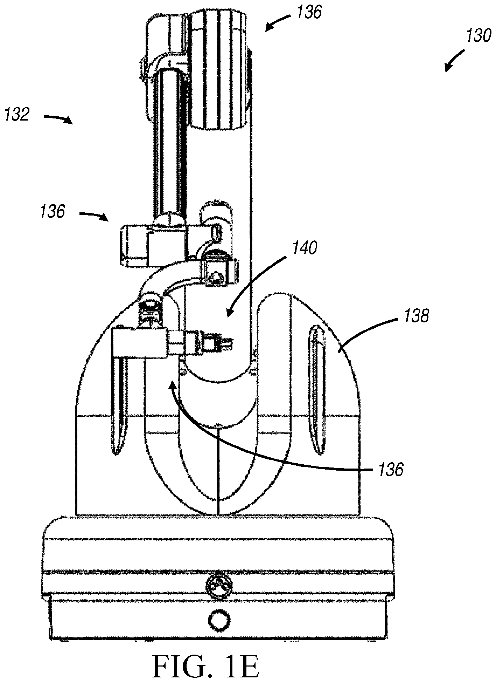

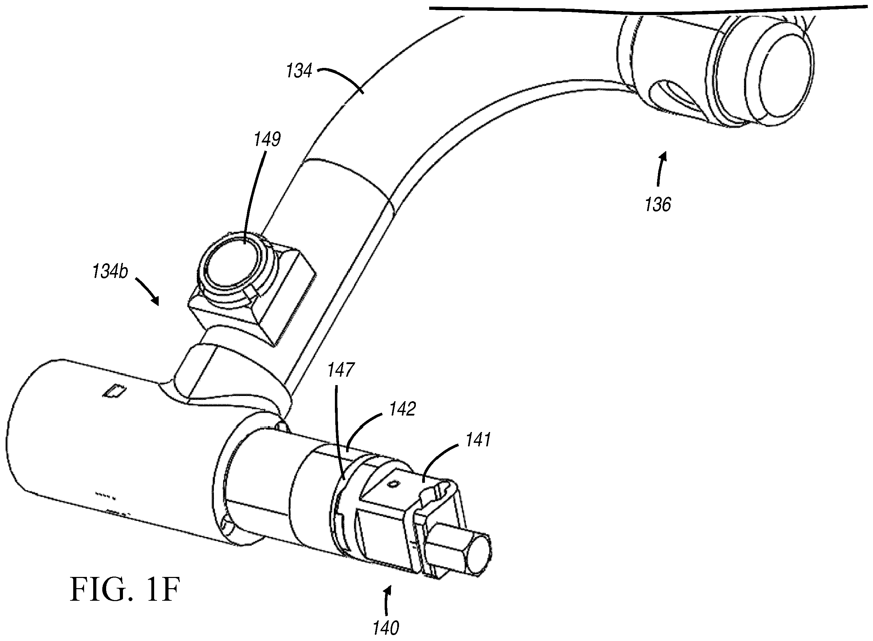

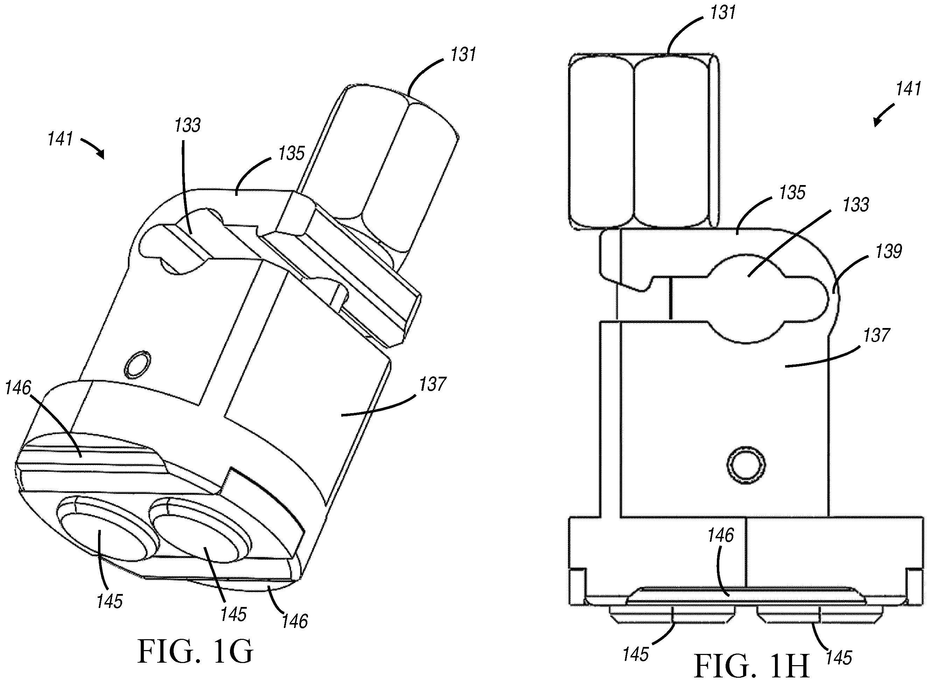

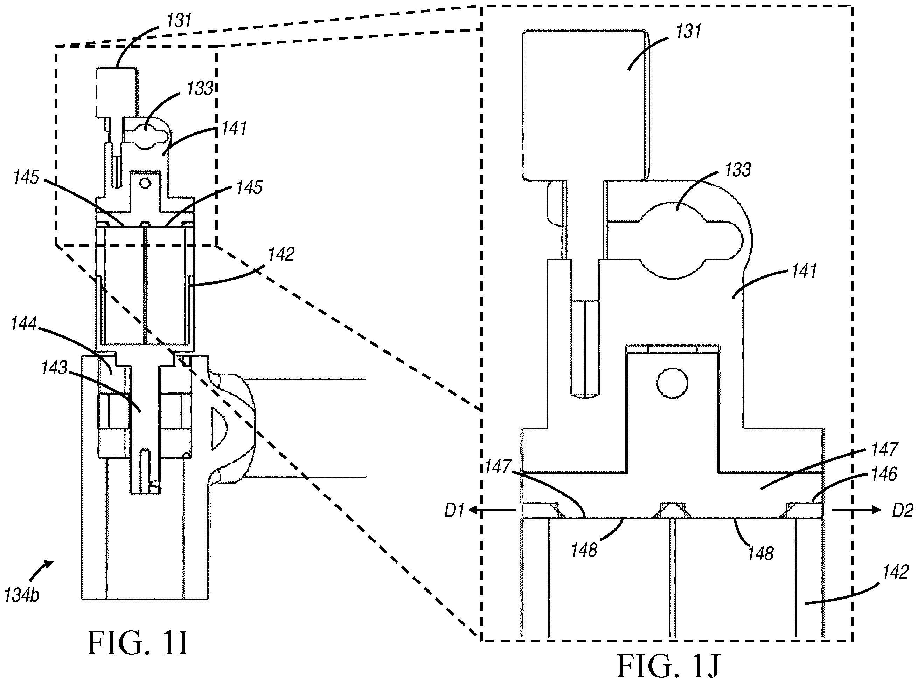

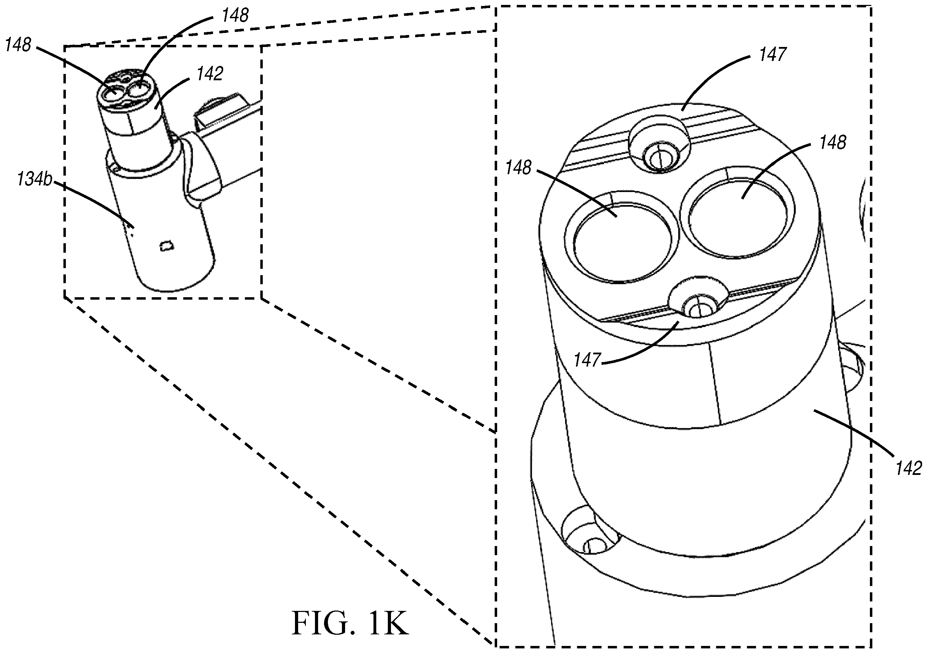

An advantage of some arrangements of the robotic system 100 disclosed herein is an increase in the accuracy of the movements or actions of the operator when the operator manipulates one or more arms of the robotic system 100 and/or one or more surgical instruments coupled with robotic arms of the robotic system 100. With reference to Figure 1, any embodiments of the robotic system 100 can have one or more arms 102 having one or more arms segments 104 and one or more articulation joints 106 that can be coupled with a base portion 108. The base portion 108 can include a frame. Any embodiments of the robotic system 100 can be configured such that the various arms 102 can be interchanged, swapped, or coupled with the base portion 108 of the robotic system 100 in any desired arrangement.

Some embodiments of the robotic system 100 can have a plurality of arms 102 that can have an interface configured to permit a range of different end effectors, which can include surgical instruments, to be removably and repeatably attached to the arms 102 of the robotic system 100. The one or more arm segments 104 can each have a proximal end portion 104a and a distal end portion 104b. An instrument coupler 110 can be coupled with a distal end portion 104b of a distal most arm segment 104. In any embodiments disclosed herein, the coupler 110 (including the coupler body or the coupler interface, described below) can be coupled with a distal end portion 104b of the arm 102 using any suitable fasteners or connectors, including without limitation, magnets, screws, pins, clamps, welds, adhesive, rivets, and/or any other suitable faster or any combination of the foregoing. For example and without limitation, in some embodiments, the coupler body can have one or more magnets and the coupler interface or an end portion of the arm of the robotic system can have a ferrous base component configured to receive and magnetically couple with the one or more magnets of the coupler body so that the coupler (e.g., the coupler body and/or the coupler interface) can be removably coupled with the end portion of the arm.

In some embodiments, the coupler interface can have one or more magnets and an end portion of the arm of the robotic system can have a ferrous base component configured to receive and magnetically couple with the one or more magnets of the coupler body or interface so that the coupler body or interface can be removably coupled with the end portion of the arm. In any embodiments, the magnets can be coupled with the end portion of the arm and can be configured to magnetically couple with a ferrous base portion of the coupler body or the coupler interface. For example and without limitation, in some embodiments, the coupler interface can have a ferrous base component and an end portion of the arm of the robotic system can have one or more magnets configured to receive and magnetically couple with the ferrous base component of the coupler interface so that the coupler can be removably coupled with the end portion of the arm. For example and without limitation, in some embodiments, the coupler interface can have one or more magnets and an end portion of the arm of the robotic system can have one or more magnets configured to receive and magnetically couple with the one or more magnets of the coupler interface so that the coupler can be removably coupled with the end portion of the arm. In embodiments in which each of the coupler interface and the end portion of the arm comprises at least two magnets, the polarity of the magnets can ensure appropriate coupling orientation. Some embodiments of the coupler 110 can be configured to releasably couple with a surgical instrument 112. The surgical instrument 112 can be any desired laparoscopic or other surgical instrument, including without limitation, a needle holder, clamp, or scissors.

In some embodiments, the coupler 110 can couple with the instrument at an attachment point, wherein the coupler 110 can include a passive ball joint at the attachment point with the instrument. Any of the embodiments of the robotic arms or other components of the robotic systems disclosed herein can include any of the components, features, or other details of any of the embodiments disclosed in U.S. Patent No. 10,582,977, which patent is hereby incorporated by reference as if fully disclosed and set forth herein. This includes, without limitation, any details of the attachment point, passive ball joint, or any other components, features, or other details disclosed in U.S. Patent No. 10,582,977, any of which can be used in combination with any of the components, features, and/or other details disclosed herein.

Any embodiments of the robotic arms disclosed herein can include one or more motors 120 at each of the articulation joints 106, the one or more motors 120 being configured to rotate the one or more arms segments 104 to manipulate the robotic arms 102. In any embodiments disclosed herein, the one or more motors 120 can be configured to produce an impedance at any of the articulation joints 106. In some embodiments, impedance can be produced at each of the articulation joints 106, the coupler 110, and/or the surgical instrument 112 to improve the sensations experienced by the operator during manipulation of the robotic arms 102, the coupler 110, and/or the surgical instrument 112 and also for improving the actions of the operator during surgical procedures. For example and without limitation, impedance can be used to provide a sensation of a viscosity, a stiffness, and/or an inertia (or a combination thereof) that can be applied to the surgical instrument 112 which the operator can feel. In some embodiments, the impedances can simulate a tissue density or stiffness, can communicate surgical boundaries to the operator, can be used to direct a surgical instrument along a desired path, or otherwise.

The one or more motors 120 can be controlled by a processing unit 122 comprising a processor configured to control the motors 120. Some embodiments of the robotic system 100 disclosed herein can have one or more encoders 124 (which can be absolute encoders) or other sensors at the articulation joints 106 of the arms of the robotic system 100 to accurately determine the position and/or angulation of the robotic arms and/or the exact position of any instruments coupled with robotic arms. In some embodiments, one or more of the robotic arms 102 can have two articulation joints 106, or three or more articulation joints 106, or four or more articulation joints 106 and a corresponding number of motors 120 and encoders 124 or other position and/or angulation sensors. With three articulation joints 106, some embodiments of the robotic arms 102 can be configured to move the coupler 110 and/or instrument 112 in three degrees of freedom. In some embodiments having three articulation joints 106, some embodiments of the robotic arms 102 can be configured to provide a force at the coupler 110 in three directions. In addition, any embodiments of the robotic system 100 can have three passive degrees of freedom at a passive ball joint positioned on an end of an arm 102 of the robotic system 100. More details regarding the passive degrees of freedom and the passive ball joint are set forth in U.S. Patent No. 10,582,977, which description is hereby incorporated by reference as if fully disclosed and set forth herein.

In any embodiments, the video monitoring that can be provided by one or more cameras and/or more endoscopes can also be controlled by or manipulated by the robotic system 100. For example and without limitation, one or more of the robotic arms 102 can be coupled with and manipulate an endoscope while another of the robotic arms 102 of the robotic system 100 can be coupled with and manipulate a surgical instrument.

With reference to Figure 1, some embodiments of a co-manipulation surgical robotic system 100 disclosed herein can have one or a plurality of arms 102 that can be used for laparoscopic and other surgical procedures. The robotic system 100 can be configured to permit an operator to manually manipulate an arm 102 and/or a surgical instrument 104 that can be coupled with an arm 102 of the robotic system 100 by exerting one or more forces directly on the arm 102 and/or the surgical instrument 104. The forces can include any combination of one or more linear forces and/or one or more torques.

In any embodiments, the connections/interfaces between the arm or arms 102 of the robotic system 100 and the surgical instrument, which can be provided by the couplers 110 in some embodiments, can have electrical connectors to produce an electronic connection between the robotic arm 102 and the surgical instrument 112. In some embodiments, the robotic system 100 can be configured to pass one or more electrical signals through the drape. Any embodiments of the robotic system 100 disclosed herein can be configured to couple with a passive, electronics free instrument wherein no electrical signal passes through the drape 133.

Any embodiments of the coupler 110 disclosed herein can be configured to move between a first, unsecured or open state or position and a second, secured or closed state or position. In some embodiments, in the first state, a surgical instrument 112 can be removed from the coupler 110 and exchanged, and/or can be moved in an axial direction relative to the coupler 110. The term axial, as used in this application, is meant to describe a direction along a longitudinal axis of the object, which may be a centerline of the object or a centerline of a tubular portion of the object. In some embodiments, in the second state, the coupler 110 can secure the surgical instrument so as to axially constrain the surgical instrument 112 such that the surgical instrument 112 is at least inhibited (e.g., prevented) from axial or, in some embodiments, axial and rotational movement when the coupler is in a secured state. In some embodiments, the coupler 110 can be configured such that, even when the coupler 110 is in the second state, the surgical instrument can freely rotate relative to the coupler 110 even though the surgical instrument can simultaneously be at least inhibited (e.g., prevented) from moving in either axial direction. In this arrangement, any embodiments of the coupler 110 can be used to selectively couple a surgical instrument 112 to the robotic arm 102.

In some embodiments, the coupler 110 can be integrated into a sterile drape used for surgical procedures. In some embodiments, the sterile drape can couple with the surgical instrument 112 and/or other components of the robotic system 100. Some embodiments of the coupler 110 disclosed herein can be configured for single-handed coupling, so that an operator can couple a surgical instrument 112 to the coupler 110 using a single hand. Any of the embodiments of the coupler 110 disclosed herein can accommodate 5 mm surgical instruments, or any other desired surgical instruments including surgical instrument used for orthopedic and trauma surgery (OTS), and can be configured to account for diametric variations and surface variations (including variations in a coefficient of friction of the surface) of the surgical instruments. The embodiments of the coupler 110 disclosed herein can be configured for multiple use or otherwise be re-processable for multiple uses.

### Sterile Drape:

In any of the examples provided herein, as shown in Figure 1B, a surgical drape 133 (e.g., a sterile surgical drape) can be passed over the arm or arms 102 of the robotic system 100. The drape can be completely closed at an end portion thereof. Some embodiments of the drape 133 can have an opening (that can have a sterile seal or interface) in a distal portion thereof that a portion of the arm 102, the coupler 110, and/or an instrument 112 can pass through. Drapes having a sealed end portion (i.e., without any openings) and being sealed along a length thereof can provide a better sterile barrier for the robotic system 100. In such examples, all of the arm 102 of the robotic system 100 can be located inside the sterile drape 133 and/or be fully enclosed within the sterile drape except for an opening which can be at a proximal end of the drape 133 (e.g., near a base 108 of the surgical robot 100), or otherwise be on the patient side of the sterile drape.

In any embodiments, the coupler 110 can have a coupler body coupleable with (e.g., removably attachable to) a coupler interface using any desired attachment mechanism, including one or more magnets, fasteners, pins, channels, and any combination of the foregoing. In some embodiments, the sterile drape pass continuously (e.g., without a hole, a slit, or any other type of opening) between the coupler body and the coupler interface such that the coupler body is on a first side of the sterile drape and the coupler interface, robotic arm, and/or other components of the robotic system 100 are on the other side of the sterile drape.

In some embodiments, the instrument and the coupler body can be passive or nonelectronic such that no electrical wires need pass through the sterile drape. In other embodiments (not shown), electrical wires or other components can pass through an opening in the sterile drape to provide a wired communication channel to electrical components that can include, for example and without limitation, memory chips for calibration, radiofrequency probes for ablation, cameras, and other electronic components.

Figures 1B-1E show an embodiment of a robotic system 130 having an arm 132 having one or more arms segments 134 and one or more articulation joints 136 that can be coupled with a base portion 138. The robotic system 130 shown in Figures 1B-1E can have two, three, or more arms 132. Any embodiments of the robotic system 130 can be configured such that the arms 132 can be interchanged, swapped, or coupled with the base portion 138 of the robotic system 130 in any desired arrangement.

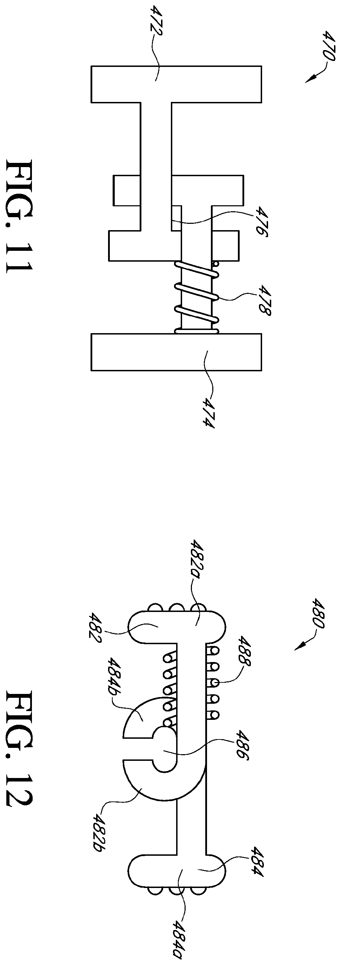

Some embodiments of the robotic system 130 can have a plurality of arms 132 that can have an interface configured to permit a range of different end effectors, which can include surgical instruments, and that can be removably attached to the arm(s) 132 of the robotic system 130. The one or more arm segments 134 can have a proximal end portion 134a and a distal end portion 134b. An instrument coupler 140 can be coupled with a distal end portion 134b of a distal most arm segment 134. In any embodiments disclosed herein, the coupler 140 (or the coupler interface 142, described below) can be coupled with a distal end portion 134b of the arm segment 134 using any suitable fasteners or connectors, including without limitation, magnets, screws, pins, clamps, or any combination of the foregoing. With reference to Figure 11, some embodiments of the coupler interface 142 can couple with a distal end portion 134b of an arm segment 134 using a fastener 143 that can be threaded or have other features that enable the fastener 143 and, hence, the coupler interface 142 to be selectively attached to the distal end portion 134b of the arm segment 134. The fastener 143 can be coupled with an insert element 144 having an opening therein to receive the fastener 143 position at or in the end portion 134b of the arm segment 134. In some embodiments, the fastener 143 can be a pin or can have other features such as a ball, a latch, or otherwise to permit the fastener 143 to selectively couple with the distal end portion 134b.

In any embodiments, the coupler body 141 be removably coupled with the coupler interface 142. For example and without limitation, the coupler body 141 can have one or more magnets 145 extending away from a surface of the coupler body 141 that, in an assembled state, contacts a surface of the coupler interface 142 or, in embodiments that do not have a coupler interface, the magnets can contact an end portion 134b of the arm segment 134. The coupler interface 142 or the end portion 134b of the arm segment 134 can have a ferrous base component configured to receive and magnetically couple with the one or more magnets 145 coupled with the coupler body 141 so that the coupler body 141 can be removably coupled with the coupler interface 142 and/or the end portion 134b of the arm segment 134. The coupler 140 can be configured so that the magnetic force can be overcome so that the coupler body 141 and the instrument 112 coupled therewith can be removed from the coupler interface 142 and/or the end portion 134b of the arm segment 134. As mentioned, some embodiments of the coupler 140 can be configured to releasably couple with a surgical instrument 112. The surgical instrument 112 can be any desired laparoscopic or other surgical instrument, including without limitation, a needle holder, clamp, or scissors.

With reference to Figures 1G-1K, some embodiments of the coupler body 141 can have one or more grooves 146 (two being shown) configured to engage with complementary raised portions or ridges 147 of the coupler interface 142. The grooves 146 and the ridges 147 can interact to assist with the alignment of the coupler body 141 with the coupler interface 142 by limiting (e.g., preventing) movement between the coupler body 141 and the coupler interface 142 in at least two directions D1 and D2, as shown in Figure 1J. In some embodiments, the coupler interface 142 can have one or more recesses or depressions 148 configured to receive the one or more magnets 145 therein. The recesses 148 can be configured to limit (e.g., prevent) movement between the coupler body 141 and the coupler interface 142 in any direction that is radial or normal to an axial (e.g., longitudinal) centerline of the magnets 145 when the coupler body 141 is in an assembled position or state with the coupler interface 142. This can further limit a movement of the coupler body 141 relative to the coupler interface 142.

The coupler body 141 can be coupled with the surgical instrument 112 at any desired axial position on the surgical instrument 112. Once the coupler body 141 is coupled with the surgical instrument 112, the coupler body 141 and the surgical instrument 112 that is coupled with the coupler body 141 can be coupled with the coupler interface 142. In some embodiments, the coupler body 141 can be configured such that, once the coupler body 141 is coupled with the surgical instrument 112, the surgical instrument 112 can be at least inhibited (e.g., prevented) from moving axially or, in some embodiments, moving axially and rotationally, relative to the coupler body 141. The coupler 140 can be configured such that the coupler body 141 can be at least inhibited (e.g., prevented) from moving in any axial direction relative to the coupler interface 142. In some embodiments, the coupler 140 can be configured such that the coupler body 141 is free to rotate relative to the coupler interface 142. In this configuration, a surgical instrument 112 coupled with the coupler body 141 can be freely rotated by an operator relative to a coupler interface 142 that the coupler body 141 is coupled with, and can be at least inhibited from (e.g., prevented from) any axial movement relative to the coupler interface 142 that the coupler body 141 is coupled with.

In other embodiments, the coupler 140 can be configured such that the surgical instrument 112 can be moved in an axial direction relative to the coupler body 141 upon the application of at least a threshold force on the surgical instrument 112 relative to the coupler body 141 or upon actuation of a release or a state change of the coupler body 141. Such actuation can be achieved in some embodiments by, for example and without limitation, pressing a button, loosening a locking screw such as locking screw 131 or other connector, moving a dial, or otherwise changing the coupler 140, the coupler body 141, and/or the coupler interface 142 from a second, secured state to a first, unsecured state. For example and without limitation, in some embodiments, the surgical instrument 112 can be axially repositioned relative to the coupler 140 by loosening a thumbscrew 131 or other hand-operated fastener or fastening mechanism such as a clamp in the coupler body 141, repositioning the surgical instrument in the desired axial position, and re-tightening the thumbscrew 131 or other hand-operated fastener or fastening mechanism.

In some embodiments, the coupler interface 142 can have a recess sized and configured to receive a raised portion or a projection of the coupler body 141. The recess can inhibit (e.g., prevent) an axial movement in at least a first and a second direction, wherein the second direction is opposite to the first direction. In any embodiments, the coupler 140 can be configured such that the surgical instrument 112 can be permitted to rotate relative to the coupler 140 or be at least inhibited (e.g., prevented) from rotational movement relative to the coupler 140. In some embodiments, a surgical drape can be pinched or clamped between the coupler body 141 and the coupler interface 142.

In some embodiments, with reference to Figure 1H, the coupler body 141 can have a first portion 135 and a second portion 137. The first portion 135 can be coupled with, or integrally formed with, the second portion 137. In some embodiments, the first portion 135 can be coupled with, or integrally formed with, the second portion 137 via a hinge 139, which can be a living hinge formed from the same material as the first and second portions 135, 137 and/or integrally formed with the first and second portions 135, 137, as in the illustrated embodiment. The first portion 135 and the second portion 137 can form a clamp that can constrict about an instrument 112 that is positioned in the recess 133 as the screw 131 (which can be a thumb screw) is tightened, to couple the instrument 112 with the coupler body 141. The screw 131 can be loosened by hand to open or expand the recess 133 so that the instrument 112 can be removed, repositioned, rotated, and/or slid, etc. The recess 133 can include a first semi-circular cutout in the first portion 135 and a second semi-circular cutout in the second portion 135. Rubber pads, sheets, bumps, O-rings, projections, or other components or features configured to grip an outside of the instrument 112 can be positioned within or adjacent to the recess 133 so as to contact an outside surface of the instrument 112 and to grip an outside surface of the instrument 112. The rubber can be a silicone rubber or any other suitable type of rubber.

In some embodiments, the device for coupling an instrument to an arm of a surgical robot can include a body configured to selectively couple with an instrument for use in a surgical operation and an interface configured to selectively couple with the body and configured to be coupled with an end portion of a robotic arm. The body can have a recess therein configured to receive the instrument and a first portion hingedly coupled with a second portion (for example, with a living hinge integrally formed with the first and second portions) so that the first portion can be moved or rotated relative to the second portion to cause the recess to expand (e.g., increase in size) or contract (decrease in size). In some embodiments, the coupler can be configured to permit an instrument coupled therewith to rotate about a longitudinal axis of the instrument relative to the device when coupled with the coupler, while inhibiting longitudinal movement of the instrument relative to the device.

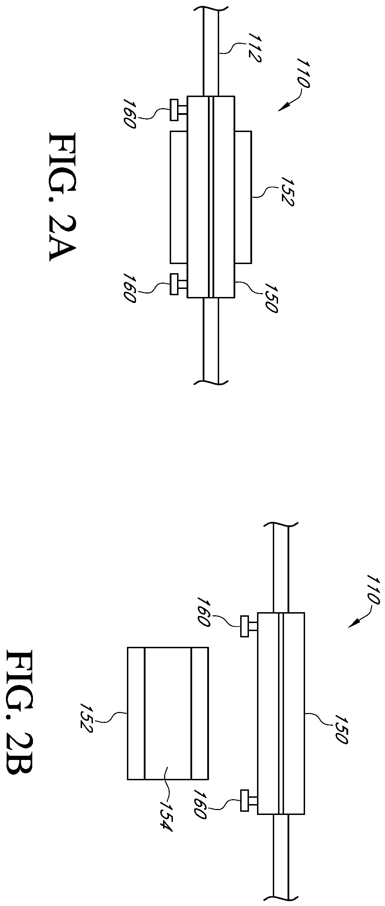

Figure 2A shows a top view of an embodiment of a coupler 110, showing a coupler body 150 (also referred to herein as a body) coupled with a coupler interface 152 (also referred to herein as an interface. Figure 2B shows a top view of the embodiment of the coupler 110 shown in Figure 2A, showing the coupler body 150 decoupled from the coupler interface 152. With reference to Figures 2A and 2B, some embodiments of the coupler 110 can have a coupler body 150 and a coupler interface 152. The coupler interface 152 can be coupled with the robotic arm 102 and can be configured such that the coupler body 150 can be removably coupled with the coupler interface 152. In this configuration, the coupler body 150 can be coupled with the surgical instrument 112 at any desired axial position on the surgical instrument 112. Once the coupler body 150 is coupled with the surgical instrument 112, the coupler body 150 and the surgical instrument 112 that is coupled with the coupler body 150 can be coupled with the coupler interface 152. In some embodiments, the coupler body 150 can be configured such that, once the coupler body 150 is coupled with the surgical instrument 112, the surgical instrument 112 can be at least inhibited (e.g., prevented) from moving axially or, in some embodiments, moving axially and rotationally relative to the coupler body 150. The coupler 110 can be configured such that the coupler body 150 can be at least inhibited (e.g., prevented) from moving in any axial direction relative to the coupler interface 152. In some embodiments, the coupler 110 can be configured such that the coupler body 150 is free to rotate relative to the coupler interface 152. In this configuration, a surgical instrument 112 coupled with the coupler body 150 can be free to rotate relative to a coupler interface 152 that the coupler body 150 is coupled with, and can be at least inhibited from (e.g., prevented from) any axial movement relative to the coupler interface 152 that the coupler body 150 is coupled with.

In other embodiments, the coupler 110 can be configured such that the surgical instrument 112 can be moved in an axial direction relative to the coupler body 150 upon the application of at least a threshold force on the surgical instrument 112 relative to the coupler body 150 or upon actuation of a release or a state change of the coupler body 150. Such actuation can be achieved in some embodiments by, for example and without limitation, pressing a button, loosening a locking screw or other connector, moving a dial, or otherwise changing the coupler 110, the coupler body 150, and/or the coupler interface 152 from a second, secured state to a first, unsecured state. For example and without limitation, in some embodiments, the surgical instrument 112 can be axially repositioned relative to the coupler 110 by loosening a thumbscrew 160 or other hand-operated fastener or fastening mechanism such as a clamp in the coupler body 150, repositioning the surgical instrument in the desired axial position, and re-tightening the thumbscrew 160 or other hand-operated fastener or fastening mechanism.

In some embodiments, the coupler interface 152 can have a recess 154 sized and configured to receive the coupler body 150. The recess 154 can be can inhibit (e.g., prevent) an axial movement or, in some embodiments, an axial and a rotational movement of the coupler body 150 relative to the coupler interface 152 while permitting free rotational movement of the coupler body 150 relative to the coupler interface 152. In any embodiments, the coupler 110 can be configured such that the surgical instrument 112 can be at least inhibited (e.g., prevented) from rotational movement relative to the coupler 110. This can be achieved by at least inhibiting (e.g., preventing) the rotational movement between the surgical instrument and the coupler 110, or between the coupler body 150 and the coupler interface 152. In some embodiments, a surgical drape can be pinched or clamped between the coupler body 150 and the coupler interface 152.

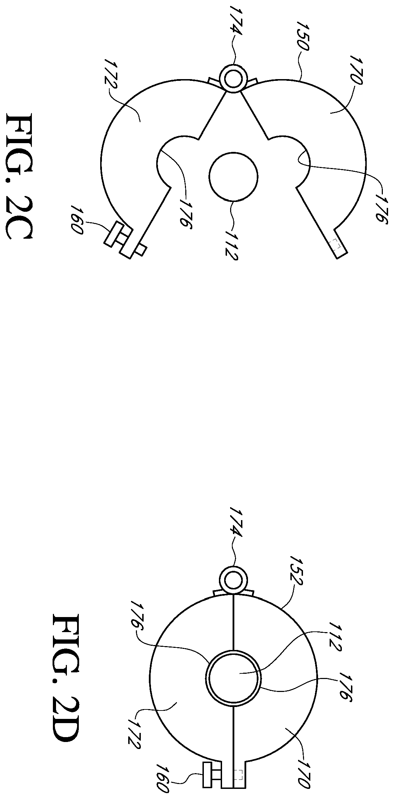

Figure 2C shows an end view of an embodiment of a coupler body 150 and a surgical instrument 112, showing the coupler body 150 in the first, unsecured or open state in which the surgical instrument 112 can be removed and replaced or repositioned relative to the coupler body 150. Figure 2D shows an end view of the embodiment of the coupler body 150 shown in Figure 2C, showing the coupler body in the second, secured or closed state in which the surgical instrument 112 can be at least inhibited (e.g., prevented) from axial movement or, in some embodiments, axial and rotational movement relative to the coupler body 150. In some embodiments, the coupler body 150 can have a first portion 170 and a second portion 172. In some embodiments, the first portion 170 can be rigidly coupled with the second portion 172 using a hinge 174 or shaft or otherwise. In some embodiments, the first and second portions 170, 172 can have a semicircular cut out or recess 176 therein sized and configured to receive the surgical instrument 112 therein. A fastener 160 can be used to couple the first half 170 with the second half 172, such as when the surgical instrument 112 is positioned in the recesses 176, as is shown in Figure 2D. As described above, the coupler body 150 can be configured to at least substantially inhibit (e.g., prevent) an axial movement or, in some embodiments, an axial and a rotational movement of the surgical instrument 112 relative to the coupler body 150. Rubber pads, sheets, bumps, O-rings, projections, or other components or features configured to grip an outside of the surgical instrument 112 can be used with any of the coupler embodiments disclosed herein. For example and without limitation, the rubber interface can be positioned within the recess or recesses of the coupler body, such as recesses 176 of the first portion 170 and/or the second portion 172 of the coupler body 150 and can be coupled to the coupler body 150. The rubber can be a silicone rubber or any other suitable type of rubber.

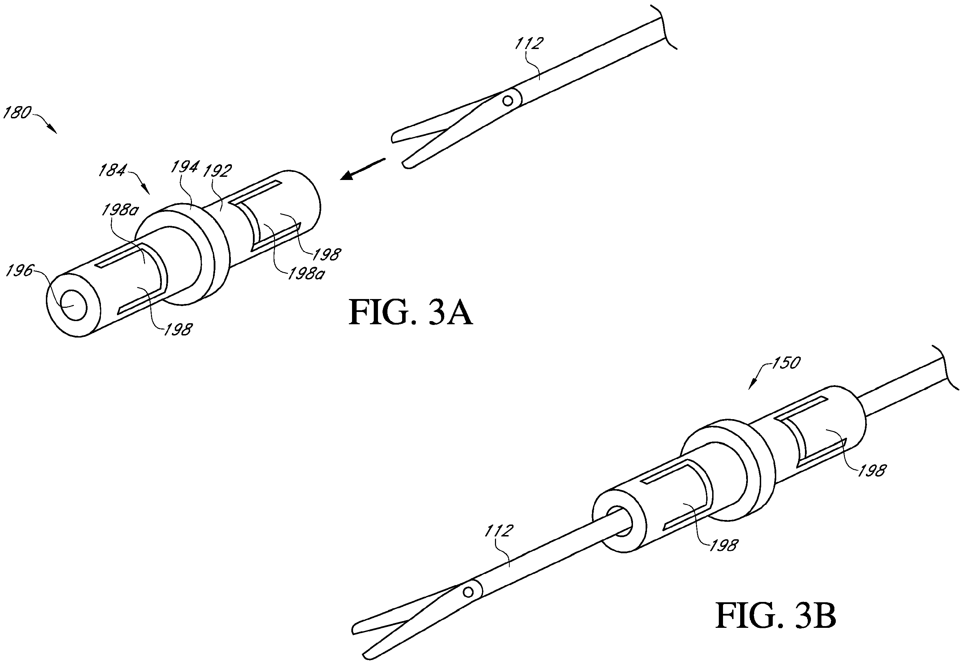

Figures 3A-3D illustrate another embodiment of a coupler 180 that can be used with any robotic system embodiments disclosed herein to couple an instrument to an end portion of a robotic arm. The coupler 180 can include a coupler body 184 and coupler interface 182 that can have a recess or depression 190 configured to receive the coupler body 184 therein. In any embodiments disclosed herein, the (including, without limitation, coupler interface 182) can be coupled with an end portion of an arm 102 of the robotic system 100. In any embodiments disclosed herein, the coupler 110 can have a coupler body 150 that removably or nonremovably couples directly with an end of an arm 102 of the robotic system 100.

With reference to Figure 3A, some embodiments of the coupler body 184 can have a cylindrical body portion 192 having an annular flange 194 projecting away from the surface of the cylindrical body portion 192. The body portion 192 can have an opening 196 extending axially through the body portion 192. The opening 196 can be sized and configured to receive a surgical instrument 112 therein. The opening 196 can be slightly larger than a diameter or outside size of the surgical instrument 112. The coupler body 184 can have one or more deflectable tabs 198 (two being shown), or four or more deflectable tabs 198 that can be configured to deflect radially inwardly so that, when the tabs 198 are deflected radially inwardly, the tabs 198 to exert a force on an outside surface of the surgical instrument 112. The coupler 180 shown in Figures 3A-3D can be configured such that, when the coupler body 184 is positioned within the recess 190 of the coupler interface 182 and the coupler interface 182 is in a second, closed or secured state, the coupler interface 182 can exert a force or otherwise deflect the tabs 198 radially inward so as to grip the surgical instrument 112 and at least inhibit (e.g., prevent) an axial movement or axial and rotational movement of the surgical instrument 112 relative to the coupler body 184. For example and without limitation, the tabs 198 can have a greater thickness near a distal end 198a of the tabs 198 such that, in a relaxed state or in the first, open state, the distal end 198a of the tabs 198 can project or protrude away from an outside surface of the body portion 192 of the coupler body 184. In this configuration, when the coupler body 184 is positioned within the recess 190 of the coupler interface 182, moving the coupler interface 182 to the second, closed state can cause a force to be applied to the distal end portions 198a of the tabs 198 to thereby deflect the tabs 198 inwardly against an outside surface of the surgical instrument 112.

In some embodiments, the recess 190 can have an enlarged portion 200 sized and configured to receive the annular flange 194 therein and to permit a rotational movement of the flange 194, while also restricting or at least inhibiting (e.g., preventing) an axial movement of the coupler body 184 by providing an axial limit to the movement of the annular flange 194. In this arrangement, the surgical instrument 112 can be axially advanced through the opening 196 of the coupler body 184 to any desired location. Thereafter, the surgical instrument 112 with the coupler body 184 coupled there with can be positioned within the recess 190 of the coupler interface 182. The coupler interface can be removably or non-removably coupled with an end portion of an arm 102 of any of the surgical robotic systems 100 disclosed herein.

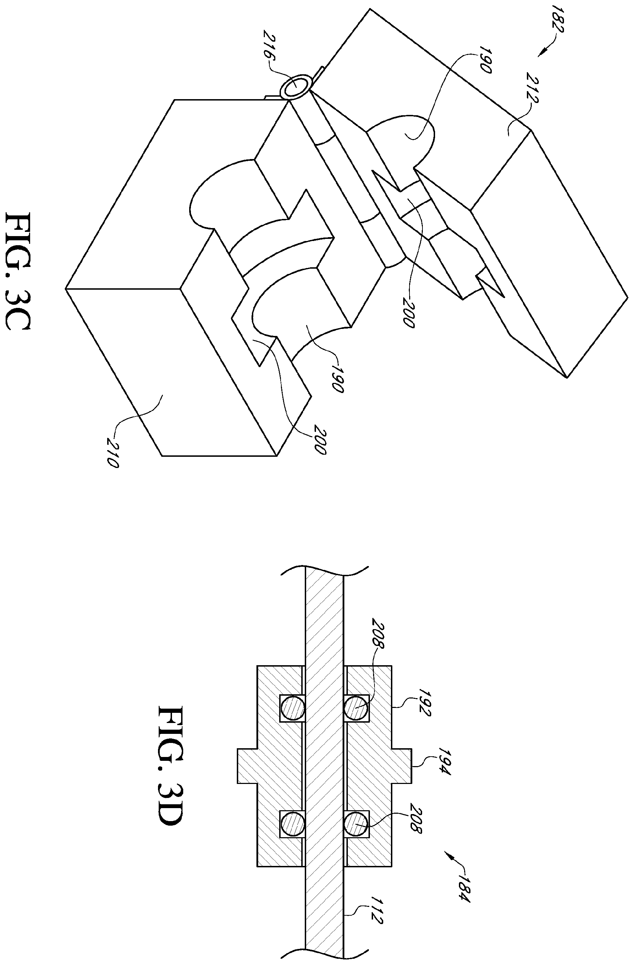

With reference to Figure 3D, rubber pads, sheets, bumps, O-rings, projections, or other gripping features 208 (O-rings being shown) configured to grip an outside of the surgical instrument 112 can be positioned within the coupler body 184 to increase a frictional force between the surgical instrument 112 and the coupler body 184. In some embodiments, the one or more tabs 198 can be configured to exert a force on the gripping features 208 when the one or more tabs 198 are deflected inwardly.

With reference to Figure 3C, some embodiments of the coupler interface 182 can have a first portion 210 that can be coupled with a second portion 212. In some embodiments, the first and second portions 210, 212 can be rigid and can be coupled to one another using a mechanical hinge 216. In other embodiments, a living hinge, a shaft, one or more fasteners, or other components or features can be used to couple the first and second portions 210, 212 together. In some embodiments, the second portion 212 can be flexible and can be configured to extend over a surgical instrument 112 and/or a coupler body 184 supported within the recess 190, such as an elastically elongatable or an elastically rigid strap. Additional fasteners, clamps, clasps, or other components or features can be used in conjunction with or in place of the hinge 216 to securely couple the first and second portions 210, 212 together once the coupler body 184 is received within the recess 190 of the coupler interface 182 to securely couple the surgical instrument 112 with the coupler 180.

In some embodiments, the coupler can include a coupler body and a coupler interface having a recess configured to receive the coupler body. The coupler body can have an opening extending axially therethrough configured to receive an instrument and an annular flange extending around an outside surface thereof. The recess in the coupler interface can have an enlarged portion configured to receive the annular flange and to permit a rotational movement of the flange while at least inhibiting (e.g., preventing) an axial movement of the coupler body by providing an axial limit to the movement of the annular flange. The coupler interface can be configured to couple with an end portion of a robotic arm.

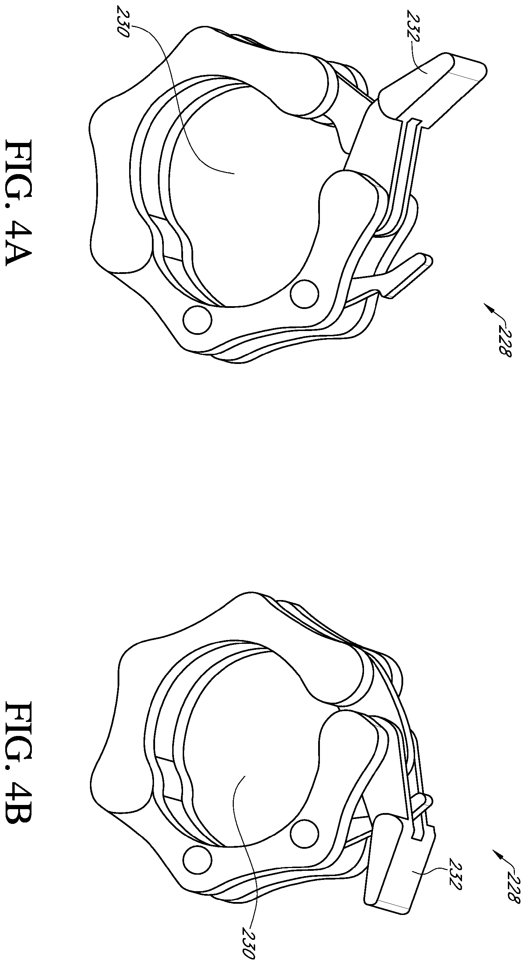

Another embodiment of a coupler body 228 is shown in Figures 4A-4B. The embodiment of the coupler body 228 shown in Figures 4A-4B can have any of the components, features, and/or other details of any of the other embodiments of the coupler body disclosed herein, in any combination with any of the components, features, and/or other details of the embodiment of the coupler body 228 shown in Figures 4A-4B. Any of the other embodiments of the coupler body disclosed herein can have any of the components, features, and/or other details of the coupler body 228 shown in Figures 4A-4B, in any combination with any of the components, features, and/or other details of the other coupler body embodiments disclosed herein.

The coupler body 228 shown in Figures 4A-4B can have an opening 230 axially therethrough configured to receive a surgical instrument therein and a clamping mechanism 232 configured to reduce an inside diameter of the opening 230 as the clamping mechanism 232 is actuated so as to cause the coupler body 228 to move from the first, unsecured or open state as shown in Figure 4A to the second, secured or closed state as shown in Figure 4B. In this arrangement, the coupler body 228 can be positioned around an outside surface of the surgical instrument while the coupler body 228 is in the first, open or unsecured state. Thereafter, the clamping mechanism 232 can be actuated so as to cause the coupler body 228 to secure itself to an outside surface of a surgical instrument. Then, the coupler body 228 can be coupled with a coupler interface sized and configured to receive and support the coupler body 228.

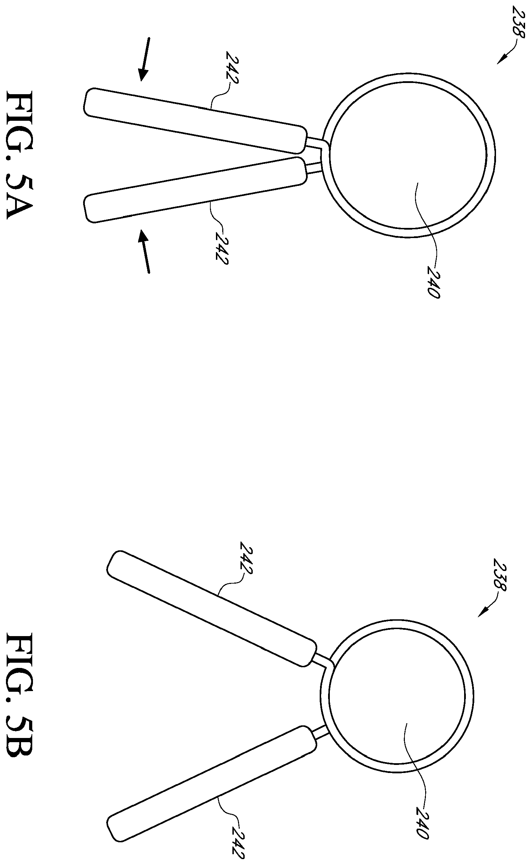

Another embodiment of a coupler body 238 is shown in Figures 5A-5B. The embodiment of the coupler body 238 shown in Figures 5A-5B can have any of the components, features, and/or other details of any of the other embodiments of the coupler body disclosed herein, in any combination with any of the components, features, and/or other details of the embodiment of the coupler body 238 shown in Figures 5A-5B. Any of the other embodiments of the coupler body disclosed herein can have any of the components, features, and/or other details of the coupler body 238 shown in Figures 5A-5B, in any combination with any of the components, features, and/or other details of the other coupler body embodiments disclosed herein.

The coupler body 238 shown in Figures 5A-5B can have an opening 240 axially therethrough configured to receive a surgical instrument therein and a clamping mechanism 242 having a first and second handle member or tab configured to reduce an inside diameter of the opening 240 as the clamping mechanism 242 is actuated so as to cause the coupler body 238 to move from the first, unsecured or open state as shown in Figure 5A to the second, secured or closed state as shown in Figure 5B. In this arrangement, the coupler body 238 can be positioned around an outside surface of the surgical instrument while the coupler body 238 is in the first, open or unsecured state. The coupler body 238 can be moved to the first, open or unsecured state by squeezing or moving the handles of the clamping mechanism 242 together, as shown in Figure 5A. Thereafter, the clamping mechanism 242 can be released so as to cause the coupler body 238 to secure itself to an outside surface of a surgical instrument. The coupler body 238 can then be coupled with a coupler interface sized and configured to receive and support the coupler body 238.

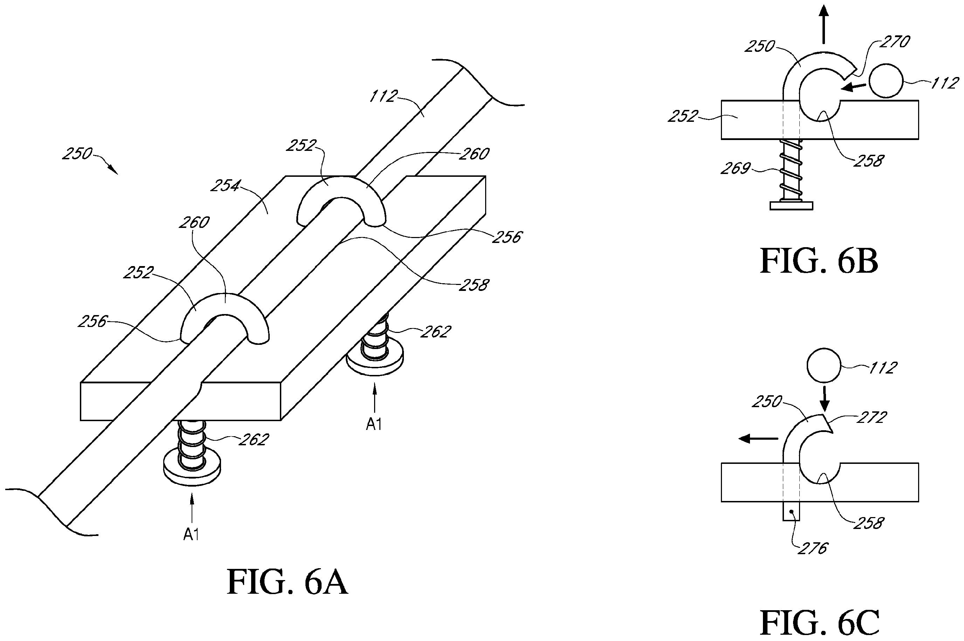

Another embodiment of a coupler 250 is shown in Figures 6A-6B. The embodiment of the coupler 250 shown in Figures 6A-6B can have any of the components, features, and/or other details of any of the other coupler embodiments disclosed herein, in any combination with any of the components, features, and/or other details of the embodiment of the coupler 250 shown in Figures 6A-6B. Any of the other coupler embodiments disclosed herein can have any of the components, features, and/or other details of the coupler 250 shown in Figures 6A-6B, in any combination with any of the components, features, and/or other details of the other coupler embodiments disclosed herein.

The coupler 250 shown in Figures 6A-6B can have one or more coupler bodies 252 (two being shown) coupled with a coupler interface 254. The coupler bodies 252 can be slidably received within openings 256 in the coupler interface 254. The coupler interface 254 can have a recess 258 which can have a semicircular cross-sectional shape or other cross-sectional shape that matches a shape of an outside surface of the surgical instrument extending along a length thereof that can be configured to receive an outside surface of the surgical instrument 112 therein. The coupler bodies can have a curved end portion 260 sized and configured to route or curve at least partially around an outside surface of the surgical instrument 112. In this configuration, the coupler bodies 252 when in a second, secured or closed position as shown in Figure 6A can be used to selectively secure the surgical instrument 112 in the recess 258 or otherwise secure the surgical instrument 112 to the coupler interface 254. Springs or other biasing mechanisms 262 can be used to bias the coupler bodies 252 in the second, closed or secured position, as shown in Figure 6A. The user can push the coupler bodies 252 in the axial direction indicated by arrow A1 so as to move the coupler bodies 252 from the second, closed or secured position to the first, open or unsecured position. The force exerted on the coupler bodies 252 should be greater than the spring or biasing force from the spring or biasing mechanisms 262 coupled with each of the coupler bodies 252.

With reference to Figure 6B, any embodiments of the coupler bodies 250 can have a sloped end surface 270. The sloped and the surface 270 can be configured such that a space between the coupler end surface 270 and an adjacent surface of the coupler interface 252 is greater at a position of coupler end surface 270 that is further away from the recess such that, as a surgical instrument 112 is advanced laterally toward a recess 258 in the coupler interface 252, an outside surface of the surgical instrument 112 can contact the end surface 270 of the coupler body and the slope of the end surface 270 of the coupler body 250 will cause the coupler body 250 to move from the second, closed or secured state toward a first, open or unsecured state to permit the surgical instrument 112 to be received within the recess 258. The coupler body 250 can have a spring or other biasing mechanism 269 configured to bias the coupler body 250 and the second, closed or secured state or position. With reference to Figure 6C, the sloped end surface 270 of any embodiments of the coupler bodies 250 can be sloped such that, as a surgical instrument 112 is advanced in a downward direction relative to an end surface 272 of a coupler body 250, such interaction between an outside surface of the surgical instrument 112 and the sloping surface 272 of the coupler body 250 can cause the coupler body 250 to rotate about a pivot point 276 away from the recess 258 and permit the surgical instrument to be received within the recess 258.

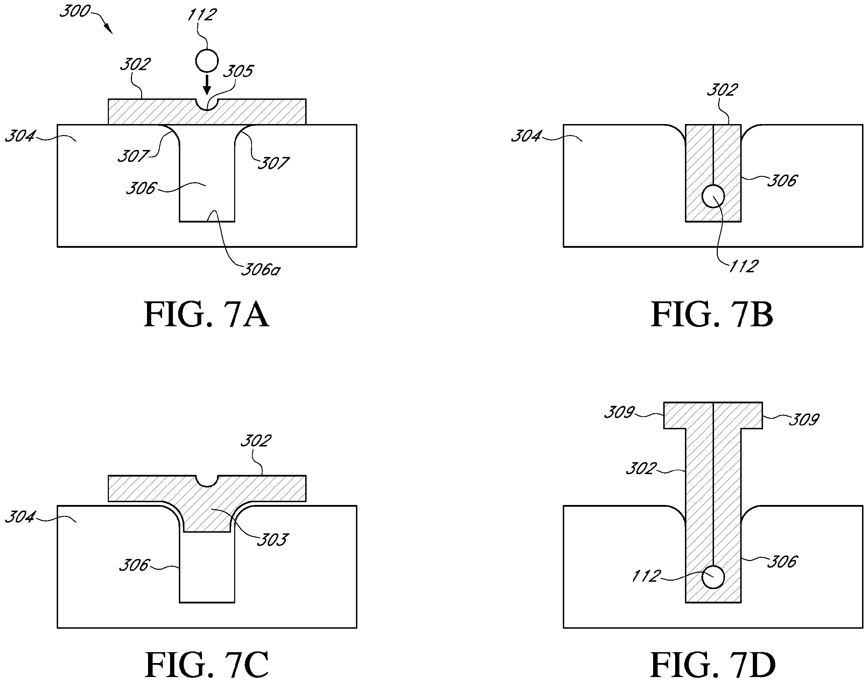

Another embodiment of a coupler 300 is shown in Figures 7A-7B. The embodiment of the coupler 300 shown in Figures 7A-7B can have any of the components, features, and/or other details of any of the other coupler embodiments disclosed herein, in any combination with any of the components, features, and/or other details of the embodiment of the coupler 300 shown in Figures 7A-7B. Any of the other coupler embodiments disclosed herein can have any of the components, features, and/or other details of the coupler 300 shown in Figures 7A-7B, in any combination with any of the components, features, and/or other details of the other coupler embodiments disclosed herein.

The coupler 300 shown in Figures 7A-7B can have a coupler body 302 that can be coupled with or engaged with a coupler interface 304. For example and without limitation, the coupler body 302 can be slidably received within a recess 306 formed in the coupler interface 304. The coupler body 302 can also have a recess 305 that can have a semicircular cross-sectional shape or other cross-sectional shape that matches a shape of an outside surface of the surgical instrument extending along a length of the coupler body 302 that can be configured to receive and at least partially surround, or in some embodiments fully surround, an outside surface of the surgical instrument 112 at least when the coupler 300 is in the second state, as shown in Figure 7B.

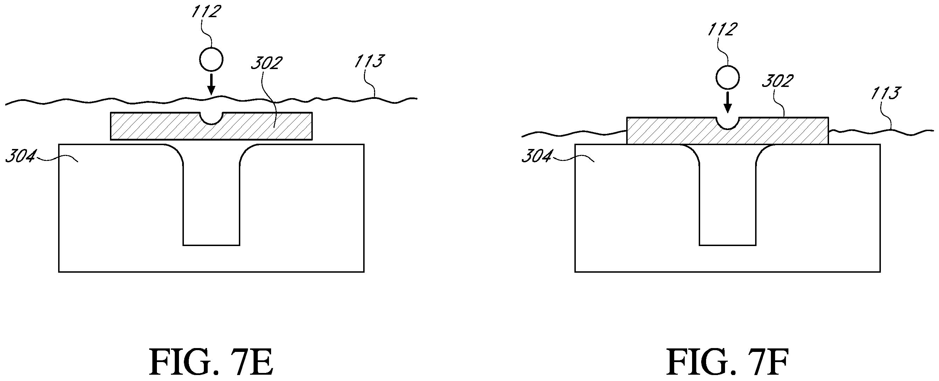

The coupler body 302 can be made from a flexible material, such as rubber including neoprene. The coupler body can have a width that is greater than a width of the recess can be biased toward a planar or generally planar shape, as shown in Figure 7A. The coupler body 302 can be flexible enough such that, when the coupler body 302 is forced toward a distal surface 306a of the recess 306, the coupler body 302 will bend or fold about a middle portion or other portion adjacent to the recess 305. Once the coupler body 302 is fully advanced into the recess 306 of the coupler interface 304, some embodiments of the coupler 300 can be configured to bias the coupler body 302 to remain within the second, secured position within the recess 306. In this configuration, to secure a surgical instrument 112 in the coupler 300, an operator can advance the surgical instrument 112 into the recess 305 of the coupler body 302, and continue to advance the surgical instrument 112 and/or the coupler body 302 toward the distal surface 306a. Some embodiments of the coupler 300 can be configured such that, once the coupler body 302 and the surgical instrument 112 have been advanced into the recess 306 of the coupler interface 304, the surgical instrument 112 will be axially and/or rotationally secured to the coupler 300. Thereafter, the coupler 300 can be coupled with an end portion of a robotic arm such that the robotic arm can be coupled with the surgical instrument 112. In any embodiments, the recess can have sloped, curved, or otherwise tapered leading edge surfaces 307 leading into the recess to facilitate the advancement of the coupler body 302 into the recess 306 of the coupler interface 304. In any embodiments, with reference to Figure 7E, a surgical drape 113 can be positioned between the surgical instrument 112 and the coupler body 302. In other embodiments, the surgical drape 113 can be integrated into the coupler body 302 so that the coupler body 302 can form a portion of the surgical drape, as shown in Figure 7F. The coupler body 302 can be flexible enough to return to the original shape of the coupler body 302 once the coupler body 302 is removed from the recess 306. In any embodiments disclosed herein, the coupler body or other components or features of the coupler can be configured to radially restrain the instrument.

With reference to Figure 7C, some embodiments of the coupler 300 can be configured such that the coupler body 302 has a projection 303 configured to extend into the recess 306 of the coupler interface 304 even when the coupler body 302 is in the first, open or unsecured state as shown in Figure 7C. the projection 303 can help bias the coupler body 302 to remain engaged with the recess 306 of the coupler interface 304 even when the coupler body 302 is in the first, open or unsecured state. With reference to Figure 7D, any embodiments of the coupler body 302 can also have protrusions, flanges, handles, tabs, or other projections 309 at a proximal end portion thereof configured to facilitate gripping and removal of the coupler body 302 from the recess 306.

In some embodiments, the coupler can include a coupler body made from a flexible material and a coupler interface having a recess configured to receive the coupler body. The coupler body can have a recess having a curved profile along a length of a first main surface thereof that is configured to receive an instrument therein. The coupler body can be flexible enough such that, when the coupler body is forced toward a distal surface of the recess, the coupler body will fold about a portion thereof adjacent to the recess, thereby at least axially and radially restraining the instrument. The coupler body can be flexible enough to return to the original shape of the coupler body 302 once the coupler body is removed from the recess.

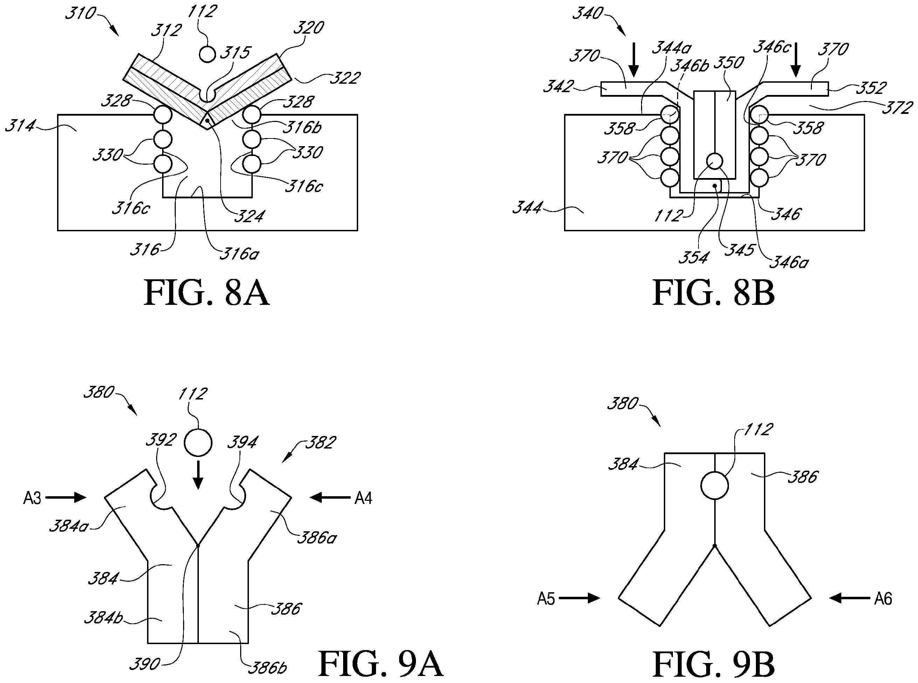

Another embodiment of a coupler 310 is shown in Figure 8A. The embodiment of the coupler 310 shown in Figure 8A can have any of the components, features, and/or other details of any of the other coupler embodiments disclosed herein, in any combination with any of the components, features, and/or other details of the embodiment of the coupler 310 shown in Figure 8A. Any of the other coupler embodiments disclosed herein can have any of the components, features, and/or other details of the coupler 310 shown in Figure 8A, in any combination with any of the components, features, and/or other details of the other coupler embodiments disclosed herein.

The coupler 310 shown in Figure 8A can have a coupler body 312 that can be coupled with or engaged with a coupler interface 314. For example and without limitation, the coupler body 312 can be received within a recess 316 formed in the coupler interface 314. The coupler body 312 can also have a recess 315 that can have a semicircular cross-sectional shape or other cross-sectional shape that matches a shape of an outside surface of the surgical instrument extending along a length of the coupler body 312 that can be configured to receive and at least partially surround, or in some embodiments fully surround, an outside surface of the surgical instrument 112 at least when the coupler 310 is in the second state.

The coupler body 312 can be made from a flexible material, such as rubber including neoprene. Other embodiments of the coupler body 312 can be made from multiple materials, including a first layer 320 made from a flexible material that can have increased gripping such as a rubber and a second layer 322 that can be a backing layer or support layer for the first layer 320 can be made from a more rigid material, such as plastic, metal, or otherwise. The recess 315 can be formed in the first layer 320. The recess 315 can be formed in a middle portion of the first layer 320. Some embodiments of the second layer 322 can have a hinge 324 in or attached to a middle portion thereof. In some embodiments, the hinge 324 can run generally parallel to the recess 315 formed in the first layer 320 and the recess 316 formed in the coupler interface 314. In some embodiments, the coupler body 312 can fold or hinge between the first, open state and the second, closed or secured state about a surgical instrument 112 by folding or hinging about the hinge 324.

The coupler body 312 can have a width that is greater than a width of the recess 316 can be. The coupler body 312 can be configured such that, when the coupler body 312 is forced toward a distal surface 316a of the recess 316, the coupler body 312 will bend or fold about the hinge 324 so as to collapse or close about the surgical instrument 112 positioned within the recess 315 of the coupler body 312 so as to secure the surgical instrument 112 within the coupler body 312 and the coupler interface 314.

Some embodiments of the coupler interface 314 can have one or more rollers 322 (two being shown) at a proximal end 316b of the recess 316 formed in the coupler interface 314. The one or more rollers 322 can facilitate the movement of the coupler body 312 into the recess 316 by permitting the coupler body 312 to roll on the rollers as the coupler body 312 is advanced into the recess 316. Some embodiments of the coupler interface 314 can have additional rollers 330 along the side wall surfaces 316c of the recess 316 to continue to facilitate the advancement of the coupler body 312 into the recess 316. In some embodiments, the recess 316 can have a generally rectangular shape. In other embodiments, the recess 316 can have a tapered or narrowing profile.

Once the coupler body 312 is fully advanced into the recess 316 of the coupler interface 314, some embodiments of the coupler 310 can be configured to bias the coupler body 312 to remain within the second, secured position within the recess 316. In this configuration, to secure a surgical instrument 112 in the coupler 310, an operator can advance the surgical instrument 112 into the recess 315 of the coupler body 312, and continue to advance the surgical instrument 112 and/or the coupler body 312 toward the distal surface 316a of the recess 316. Some embodiments of the coupler 310 can be configured such that, once the coupler body 312 and the surgical instrument 112 have been advanced into the recess 316 of the coupler interface 314, the surgical instrument 112 will be axially and/or rotationally secured to the coupler 310. Thereafter, the coupler 310 can be coupled with an end portion of a robotic arm such that the robotic arm can be coupled with the surgical instrument 112.

Another embodiment of a coupler 340 is shown in Figure 8B. The embodiment of the coupler 340 shown in Figure 8B can have any of the components, features, and/or other details of any of the other coupler embodiments disclosed herein, in any combination with any of the components, features, and/or other details of the embodiment of the coupler 340 shown in Figure 8B. Any of the other coupler embodiments disclosed herein can have any of the components, features, and/or other details of the coupler 340 shown in Figure 8B, in any combination with any of the components, features, and/or other details of the other coupler embodiments disclosed herein.

The coupler 340 shown in Figure 8B can have a coupler body 342 that can be coupled with or engaged with a coupler interface 344. The coupler body 342 can be received within a recess 346 formed in the coupler interface 344. The coupler body 342 can also have a recess 345 that can have a semicircular cross-sectional shape or other cross-sectional shape that matches a shape of an outside surface of the surgical instrument extending along a length of the coupler body 342 that can be configured to receive and at least partially surround, or in some embodiments fully surround, an outside surface of the surgical instrument 112 at least when the coupler 340 is in the second state, as shown in Figure 8B.

The coupler body 342 can be made from multiple materials, including a first layer 350 made from a flexible material that can have increased gripping such as a rubber and a second layer 352 that can be a backing layer or support layer for the first layer 350 can be made from a more rigid material, such as plastic, metal, or otherwise. The recess 345 can be formed in the first layer 350. In some embodiments, the recess 345 can be formed in a middle portion of the first layer 350. Some embodiments of the second layer 352 can have a hinge 354 in or attached to a middle portion thereof. In some embodiments, the hinge 354 can run generally parallel to the recess 345 formed in the first layer 350 and the recess 346 formed in the coupler interface 344. In some embodiments, the coupler body 342 can fold or hinge between the first, open state and the second, closed or secured state about a surgical instrument 112 by folding or hinging about the hinge 354.

The coupler body 342 can have a width that is greater than a width of the recess 346 can be. The coupler body 342 can be configured such that, when the coupler body 342 is forced toward a distal surface 346a of the recess 346, the coupler body 342 will bend or fold about the hinge 324 so as to collapse or close about the surgical instrument 112 positioned within the recess 345 of the coupler body 342 so as to secure the surgical instrument 112 within the coupler body 342 and the coupler interface 344. In some embodiments, the second layer 352 can have wings or tabs 370 that can be used to facilitate removal of the coupler body 342 from the recess 346. The tabs 370 can be formed such that, when the coupler body 342 is in the second position, as shown in Figure 8B, the tabs 370 can be spaced apart from a first surface 344a (which can be an upper surface when the coupler interface 344 is positioned as shown in Figure 8B) such that a gap or space 372 exists between the tabs 370 and the upper surface 344a of the coupler interface 344. The space 372 can be large enough to permit the tabs 370 to move toward the first surface 344a when a force is applied to the tabs 370 in the direction of the first surface 344a. As the tabs 370 are deflected toward the first surface 344a, such movement of the tabs 370 can force a remainder of the coupler body 342 to move away from a distal surface 346a of the recess 346, thereby allowing the coupler body 342 to be removed from the recess 346.

Some embodiments of the coupler interface 344 can have one or more rollers 358 (two being shown) at a proximal end 346b of the recess 346 formed in the coupler interface 344. The one or more rollers 358 can facilitate the movement of the coupler body 342 into the recess 346 by permitting the coupler body 342 to roll on the rollers as the coupler body 342 is advanced into the recess 346. Some embodiments of the coupler interface 344 can have additional rollers 370 along the side wall surfaces 346c of the recess 346 to continue to facilitate the advancement of the coupler body 342 into the recess 346.

Once the coupler body 342 is fully advanced into the recess 346 of the coupler interface 344, some embodiments of the coupler 340 can be configured to bias the coupler body 342 to remain within the second, secured position within the recess 346. In this configuration, to secure a surgical instrument 112 in the coupler 340, an operator can advance the surgical instrument 112 into the recess 345 of the coupler body 342, and continue to advance the surgical instrument 112 and/or the coupler body 342 toward the distal surface 346a of the recess 346. Some embodiments of the coupler 340 can be configured such that, once the coupler body 342 and the surgical instrument 112 have been advanced into the recess 346 of the coupler interface 344, the surgical instrument 112 will be axially and/or rotationally secured to the coupler 340. Thereafter, the coupler 340 can be coupled with an end portion of a robotic arm such that the robotic arm can be coupled with the surgical instrument 112.

Another embodiment of a coupler 380 is shown in Figure 9A. The embodiment of the coupler 380 shown in Figure 9A can have any of the components, features, and/or other details of any of the other coupler embodiments disclosed herein, in any combination with any of the components, features, and/or other details of the embodiment of the coupler 380 shown in Figure 9A. Any of the other coupler embodiments disclosed herein can have any of the components, features, and/or other details of the coupler 380 shown in Figure 9A, in any combination with any of the components, features, and/or other details of the other coupler embodiments disclosed herein.

The coupler 380 shown in Figure 9A can have a coupler body 382 that can be coupled with or engaged with a coupler interface (not shown) or can be coupled with or engaged with a robotic arm without the presence of a coupler interface (e.g., the coupler body of any embodiments disclosed herein can directly engage or interface with an end portion of the robotic arm). The coupler body 382 can have a first portion 384 and a second portion 386 coupled with the first portion 384. In some embodiments, the first portion 384 can be hingedly or rotatably coupled with the second portion 386. For example and without limitation, the coupler body 382 can have a hinge or joint 390 that can couple the first and second portions 384, 386 together.

In some embodiments, the first portion 384 of the coupler body 382 can have a proximal portion 384a and a distal portion 384b that is integrally formed with or coupled with the proximal portion 384a. The first portion 384 of the coupler body 382 can have a recess 392 and the second portion 386 of the cover body 382 can have a recess 394, each of which can have a semicircular cross-sectional shape or other cross-sectional shape that matches a shape of an outside surface of the surgical instrument extending along a length of the coupler body 382 that can be configured to receive and at least partially surround, or in some embodiments fully surround, an outside surface of the surgical instrument 112 at least when the coupler 382 is in the second state. The second state of the coupler body 382 is shown in Figure 9B. In some embodiments, the second portion 386 can be similarly situated and can be a mirror copy of the first portion 384, with a proximal portion 386a and a distal portion 386b that is integrally formed with or coupled with the proximal portion 386a.

Some embodiments of the coupler 380 can be configured to be bistable in that the coupler 380 will be biased toward either the first, open or unsecured state or the second, closed or secured state and is unstable in any position or state except the first and second states. In the first state, the distal portion 384b of the first portion 384 of the coupler 380 is in contact with the distal portion 386b of the second portion 386 of the coupler 380 and the proximal portion 384a of the first portion 384 of the coupler 380 is rotated away and spaced apart from the proximal portion 386a of the second portion 386 of the coupler 380. In the first, open or unsecured state, a surgical tool 112 can be loaded into or removed from the coupler 380. In the second state, the proximal portion 384a of the first portion 384 of the coupler 380 is in contact with the proximal portion 386a of the second portion 386 of the coupler 380 and the distal portion 384b of the first portion 384 of the coupler 380 is rotated away and spaced apart from the distal portion 386b of the second portion 386 of the coupler 380. In the second, closed or secured state, the surgical tool 112 loaded into the coupler 380 can be secured or supported by the coupler 380 such that the surgical instrument 112 can be at least inhibited (e.g., prevented) from an axial movement or, in some embodiments, an axial and a rotational movement relative to the coupler 380.

In this configuration, when the coupler 380 is in the first, open state as shown in Figure 9A, after positioning a surgical instrument 112 in either the recess 392 with the recess 394, the operator can change the coupler 380 to the second, closed state by pinching or moving the proximal portion 384a of the first portion 384 toward the proximal portion 386a of the second portion 386, such as by exerting a force on the proximal portions 384a, 386a of the first and second portions 384, 386 along the directions A3 and A4, as shown in Figure 9A (e.g., by squeezing the proximal portions 384a, 386a of the first and second portions 384, 386 together). When the coupler 380 is in the second, closed state as shown in Figure 9B, the operator can change the coupler 380 to the first, open state by pinching or moving the distal portion 384b of the first portion 384 toward the distal portion 386b of the second portion 386, such as by exerting a force on the distal portions 384b, 386b of the first and second portions 384, 386 along the directions A5 and A6, as shown in Figure 9B (i.e., by squeezing the distal portions 384b, 386b of the first and second portions 384, 386 together).

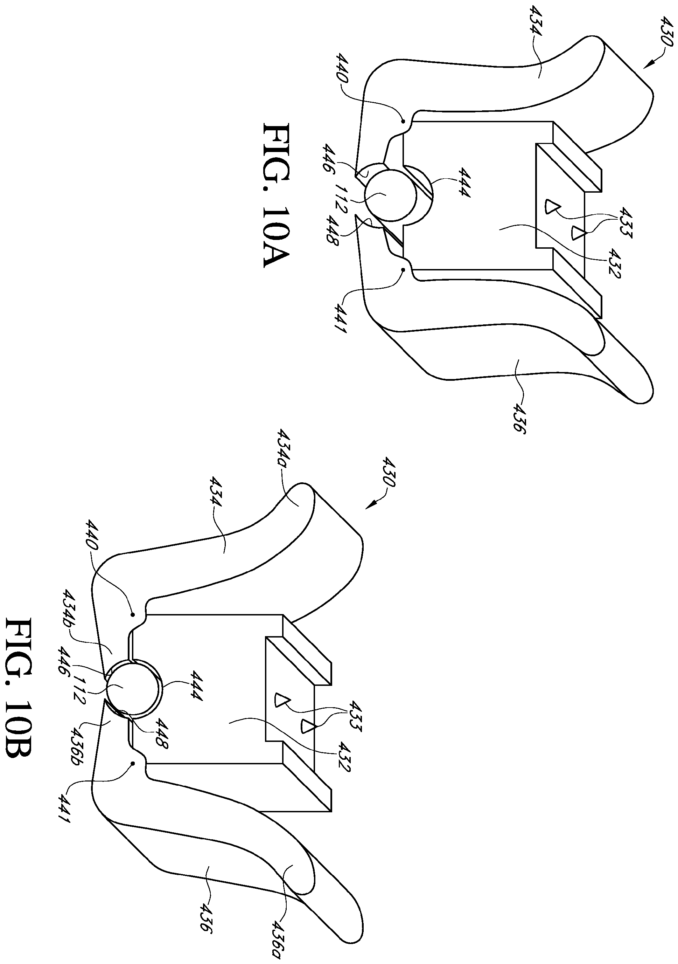

Another embodiment of a coupler 430 is shown in Figure 10A. The embodiment of the coupler 430 shown in Figure 10A can have any of the components, features, and/or other details of any of the other coupler embodiments disclosed herein, in any combination with any of the components, features, and/or other details of the embodiment of the coupler 430 shown in Figure 10A. Any of the other coupler embodiments disclosed herein can have any of the components, features, and/or other details of the coupler 430 shown in Figure 10A, in any combination with any of the components, features, and/or other details of the other coupler embodiments disclosed herein.

The coupler 430 shown in Figure 10A can have a coupler body 432 that can be coupled with or engaged with a coupler interface (not shown) or can be coupled with or engaged with a robotic arm without the presence of a coupler interface. The coupler body 432 can have one or more projections 433 (two being shown) that can be used to center or position the coupler body 432 relative to the coupler interface. For example without limitation, the projections 433 can be conical projections configured to engage with depressions or openings in the coupler interface to align the coupler body 432 with the coupler interface. In some embodiments, the coupler interface can have an equal number or a different number of depressions or openings as compared to the number of projections 433. In other embodiments, the projections 433 can be cylindrically shaped. In some embodiments, the coupler body 432 can have three or more projections 433.

The coupler body 432 can have a first tab 434 hingedly or rotatably coupled with the coupler body 432 and a second tab 436 hingedly or rotatably coupled with the coupler body 432. For example and without limitation, the coupler body 432 can have a first hinge or joint 440 that can couple the first tab 434 with the coupler body and a second hinge or joint 441 that can couple the second tab 436 with the coupler body. The first tab 434 can have a proximal end portion 434a and a distal end portion 434b. The second tab 436 can have a proximal end portion 436a and a distal end portion 436b.

The coupler body 432 can have a recess 444 formed therein, the first tab 434 can have a recess 446 formed in a distal end portion thereof and the second tab 436 can have a recess 448 formed in a distal end portion thereof, each of which can have a semicircular cross-sectional shape or other cross-sectional shape that, all together, can match a shape of an outside surface of the surgical instrument extending along a length of the coupler body 432, the first tab 434, and the second tab 436 and that can be configured to receive and at least partially surround, or in some embodiments fully surround, an outside surface of the surgical instrument 112 at least when the coupler 432 is in the second state. The second state of the coupler body 432 is shown in Figure 10B. In some embodiments, the second tab 436 can be similarly situated and can be a mirror copy of the first tab 434.

Some embodiments of the coupler 430 can be biased toward the second state, using springs or other torsional biasing elements. An operator can overcome the bias or otherwise move the coupler body 432 from the second state as shown in Figure 10B to the first state as shown in Figure 10A by squeezing together or toward one another the proximal end portions 434a, 436a of the first and second tabs 434, 436. In the first state, the operator can remove the surgical instrument 112 from the coupler 430. To support a surgical instrument 112 in the coupler 430, while the coupler 430 is in the first, open state, the operator can position the surgical instrument 112 in contact with or near the recess 444 and release the force that was applied to the first and second tab 434, 436 or otherwise relax the first and second tab 04 34, 436 and allow the first and second tabs 434, 436 to return to the relaxed position of the first and second tabs 434, 436.

Figures 11 - 14 show additional embodiments of a coupler 470, 480, 490. The embodiments of the couplers 470, 480, 490 can have any of the components, features, and/or other details of any of the other coupler embodiments disclosed herein, in any combination with any of the components, features, and/or other details of the embodiment of the couplers 470, 480, 490. Any of the other coupler embodiments disclosed herein can have any of the components, features, and/or other details of the couplers 470, 480, 490 in any combination with any of the components, features, and/or other details of the other coupler embodiments disclosed herein.

The coupler 470 shown in Figure 11 can have a first body portion 472 and a second body portion 474 that can be slidably coupled with or engaged with the first body portion 472. The coupler 470 can have a recess or opening 476 that can be enlarged and can be configured to receive a surgical instrument 112 therein when the second body portion 474 is moved toward the first body portion 472. A spring or other biasing mechanism 478 can be used to bias the coupler 470 toward the second, closed or secured state so that, when an operator releases the first and second body portions 472, 474, the coupler 470 can exert a force on a surgical instrument to secure the surgical instrument therein. Some embodiments of the coupler 470 can be figured to axially restrain a surgical instrument therein, but to permit a rotation of the surgical instrument. The coupler 470 can be coupled with a coupler interface or directly to an end portion of a surgical arm.

The coupler 480 shown in Figure 12 can have a first body portion 482 and a second body portion 484 that can be slidably coupled with or engaged with the first body portion 482. The coupler 480 can have a recess or opening 486 that can be enlarged and can be configured to receive a surgical instrument 112 therein when the second body portion 484 is moved toward the first body portion 482. A spring 488 or other biasing mechanism can be used to bias the coupler 480 toward the second, closed or secured state so that, when an operator releases the first and second body portions 482, 484, the coupler 480 can exert a force on a surgical instrument to secure the surgical instrument therein. Some embodiments of the coupler 480 can be figured to axially restrain a surgical instrument therein, but to permit a rotation of the surgical instrument. The coupler 480 can be coupled with a coupler interface or directly to an end portion of a surgical arm.

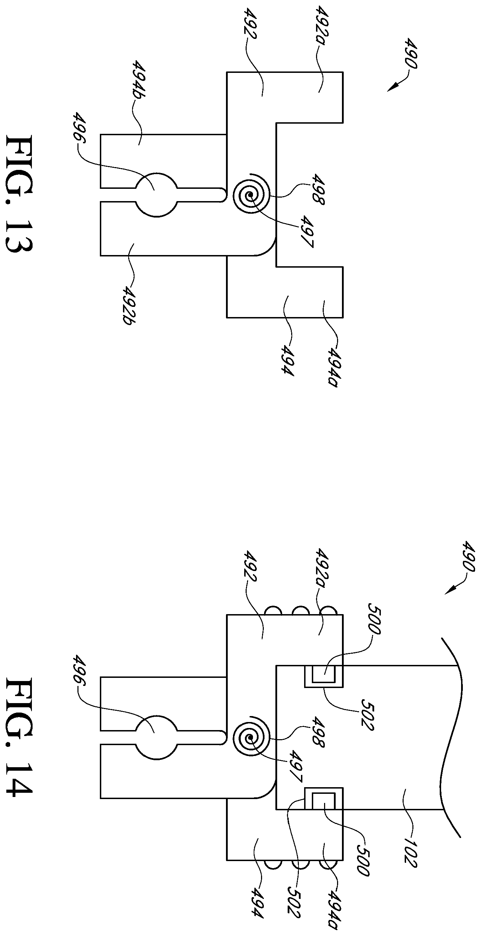

The coupler 490 shown in Figure 13 can have a first body portion 492 having a proximal end portion 492a and a distal end portion 492b and a second body portion 494 having a proximal end portion 494a and a distal end portion 494b that can be rotatably coupled with or engaged with the first body portion 492 about an axis or shaft 497. The coupler 490 can have a recess or opening 496 formed in the distal end portions 492b, 494b that can be enlarged and can be configured to receive a surgical instrument 112 therein when the distal end portion 494b of the second body portion 494 is rotated away from the distal end portion 492b of the first body portion 492. A spring 498 or other biasing mechanism can be used to bias the coupler 490 toward the second, closed or secured state so that, when an operator releases the first and second body portions 492, 494, the coupler 490 can exert a force on a surgical instrument to secure the surgical instrument therein. Some embodiments of the coupler 490 can be figured to axially restrain a surgical instrument therein, but to permit a rotation of the surgical instrument. The coupler 490 can be coupled with a coupler interface or directly to an end portion of a surgical arm.

With reference to Figure 14, some embodiments of the coupler can be configured to engage with a coupler interface or the distal end portion of a robotic arm member 102. For example without limitation, some embodiments of the coupler 490 can have projections 500 extending inwardly from an inner surface of the proximal end portion 492a of the first body portion 492 and an inner surface of the proximal end portion 494a of the second body portion 494 that can be received within recesses 502 formed in a distal end portion of the robotic arm 102 when the coupler 490 is in the second, closed state.

Any embodiments of the robotic system 100 disclosed herein can also or alternatively use collects, Swagelok components, Tuohy valve components, and/or other locking or constricting components to couple the surgical instrument to the robotic arm 102. In some embodiments, a length of the couple can be approximately 10 mm, or from 10 mm or less to 30 mm, or from 10 mm to 15 mm. Any of the embodiments of the coupler disclosed herein can be sized and configured to work with any desired or available surgical instrument, including surgical instruments having a 3 mm, 5 mm, 8 mm, and 10 mm outer diameter. In some embodiments, a ferrule can be used to accommodate the various diameters of the outside surface of the surgical instruments.

### Position on Surgical Instrument

Any embodiments of the coupler disclosed herein can also be configured to be secured at any desired location along a length of the surgical instrument 112. Because, in some embodiments, the position of the coupler can be varied along the length of the surgical instrument 112, some embodiments of the robotic system 100 can be configured to determine the axial position of the coupler along the length of the surgical instrument 112. This can help the robotic system 100 determine a length of the surgical instrument 112 that extends on either side of the coupler, the position of a distal portion or distal tip of the surgical instrument 112, and or the position of a proximal portion or proximal end of the surgical instrument 112.

### Tool Identification

In some embodiments, the robotic system 100 can be configured to identify the particular type of surgical instrument that is attached to the arm 102 of the robotic system 100. Some embodiments of the robotic system 100 can be configured such that the identification of the surgical instrument can be input manually by an operator of the robotic system 100 or such that the robotic system 100 can determine the identity of the surgical instrument and, in some embodiments, other characteristics or operating parameters of the instrument such as, but not limited to, the mass of the instrument, the distance of the center of mass of the instrument relative to the attachment point of the instrument, the distance of the tip of the instrument relative to the attachment point of the instrument, or the coordinates of the center of mass of the instrument relative to the attachment point of the instrument, without any additional input from the operator of the robotic system 100.

Some embodiments can use an RFID transmitter chip (e.g., placing an RFID sticker or transmitter on the surgical instrument that can transmit information about the surgical instrument to a receiver of the robotic system 100), a near field communication (NFC) device, including but not limited to a near field magnetic induction communication device, a barcode on the surgical instrument readable by a scanner or other optical device on the robotic system 100, a magnet based or other electronic communication device, a Bluetooth transmitter, or any other suitable wireless transmitter or other suitable communication device in the surgical instrument that can communicate with a receiver in the robotic system 100 or in a control station to identify the particular surgical instrument. Some embodiments can use a weight of the surgical instrument as measured by forces that are experienced or sensed by the robotic system 100 upon coupling of the surgical instrument to the robotic system 100 to identify the surgical instrument. In some embodiments, a lookup table or other data stored in a machine-readable memory device or otherwise in the robotic system 100 or other device in communication with the robotic system 100 can be used to provide one or more parameters associated with the identified surgical instrument. The robotic system 100 can be configured to optimize the support, movement, and other performance characteristics of the arm 102 with the identified surgical instrument based on any of a desired range of parameters associated with the identified surgical instrument. Such parameters can include, without limitation, weight, dimensions, position of the center of gravity of the surgical instrument, etc.

In some embodiments, the robotic system 100 can be configured to determine the identity of the surgical instrument 112 without using wireless communication devices or sensors. For example and without limitation, the robotic system 100 can determine the identity of the surgical instrument coupled with the robotic arm 102 using information such as a weight of the surgical instrument, a rotational inertia of the surgical instrument, or other physical properties of the surgical instrument. In some embodiments, the weight of the surgical instrument can be estimated from motor currents in the surgical arm 102. In some embodiments, the weight of the surgical instrument can be estimated from sensors positioned in a distal end portion of the robotic arm 102 and/or sensors positioned in the coupler to which the surgical instrument is attached or coupled. As mentioned, in some embodiments, without limitation, a rotational inertia of the surgical instrument can be used to identify the precise surgical instrument coupled with the robotic arm 102. With the surgical instrument coupled with the coupler or the distal end portion of the robotic arm, the robotic arm can rotate and/or shaped the surgical instrument at a safe distance away from a patient or operator and use motor currents in the surgical arm 102 and or sensors in the surgical arm and or coupler to estimate the rotational inertia of the surgical instrument.

Based on the information gathered from the physical properties of the surgical instrument 112, some embodiments of the robotic system 100 can use a lookup table or other database to identify other properties or characteristics of the surgical instrument that is coupled with the robotic arm 102, such as center of gravity, length or other dimensions, device type, and or other details that can be used to properly control and manipulate the surgical instrument.

In some embodiments, magnets, reed switches, or other devices can be used to identify when a surgical instrument is coupled with the robotic arm 102 and/or the coupler interface. In any embodiments disclosed herein, the various components of the embodiments of the coupler disclosed herein can be configured to communicate one or more electrical signals through one or more of the components of the coupler.

In some embodiments, the robotic system 100 can be configured to perform a weight check of the coupler (for example, using sensors and/or motor currents) to determine if a surgical instrument is coupled with the coupler, or to perform a redundancy check to ensure the surgical instrument attached to the coupler is the surgical instrument that has been identified such as by using the NFC, RFID, or barcode.

### Removal Force

In any embodiments disclosed herein, the coupler can be configured such that the surgical instrument coupled with the coupler can be removed from the robotic arm and/or the coupler interface coupled with the robotic arm when a threshold force is applied to the surgical instrument and/or the coupler body. For example and without limitation, where one or more magnets are used to provide a biasing force to bias the surgical instrument to the coupler, to bias the surgical instrument and the coupler body that is coupled with the surgical instrument to the coupler interface, a force greater than the attraction force provided by the one or more magnets in a direction opposing the force provided by the one or more magnets must be exerted on the surgical instrument and/or the coupler body that is coupled with the surgical instrument and magnetically attracted to the coupler interface that exceeds the attraction force provided by the one or more magnets.

For example and without limitation, in any embodiments of the robotic system 100 disclosed herein, the coupler can be configured to provide 45 Newtons, or approximately 45 Newtons, of force to the surgical instrument such that a removal force of 45 Newtons or approximately 45 Newtons must be exerted on the surgical instrument in a direction opposite to the direction of the attraction or retention force being exerted on the surgical instrument by the coupler. In some embodiments of the robotic system 100 disclosed herein, the coupler can be configured to provide from 30 Newtons (or approximately 30 Newtons, or less than 30 Newtons) to 60 Newtons (or approximately 60 Newtons, or more than 60 Newtons) of force to the surgical instrument, or of any value or range within the foregoing range. In some embodiments, a button or a switch can be activated to cause the surgical instrument to be more easily removed from the robotic arm and/or the coupler interface coupled with the robotic arm. In some embodiments, actuating the button or switch can cause the attraction force between the surgical instrument and the coupler interface or the robotic arm to be significantly reduced (such as by 75%, or by from at least 50% to 85%) or eliminated.

### Operational Modes

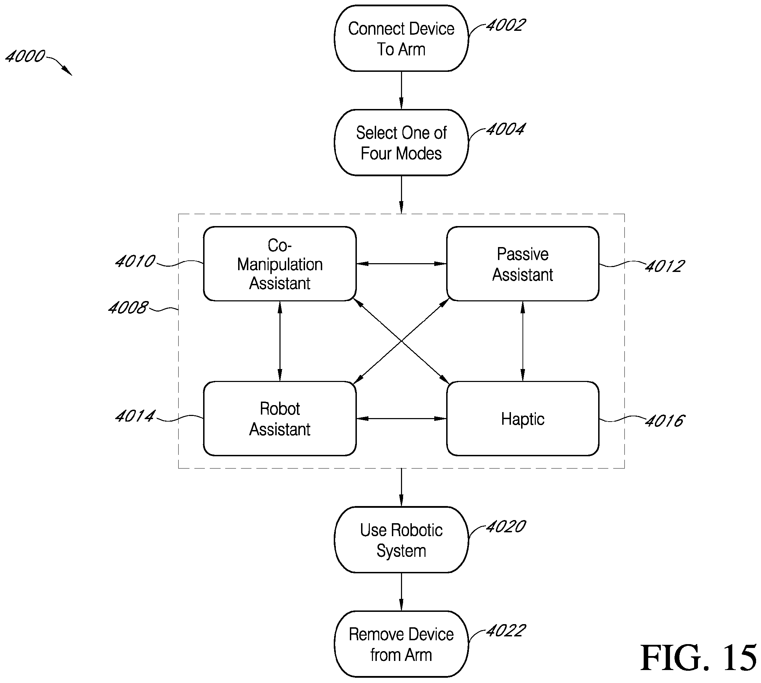

Figure 15 shows a schematic illustration of an embodiment of a mode of operation 4000 of any of the embodiments of the robotic system disclosed herein (including, for example and without limitation, robotic system 100 and robotic system 130) and/or any one or more of the arms (including, for example and without limitation, arms 102 and arms 132 of the robotic system. With reference to Figure 15, some embodiments of the robotic system can be configured to switch between at least the following operational modes 4008: Passive Co-Manipulation Assistant mode 4010 (also referred to herein as the "Co-Manipulation Assistant" mode), Passive Assistant mode 4012, Robot Assistant mode 4014, and/or Haptic mode 4016 (also referred to herein as the "Co-Manipulated Active Instrument Holder" mode). The foregoing four modes are referred to hereinafter collectively as the "operational modes". Any embodiments of the robotic system disclosed herein can be configured to switch from any one of the operational modes to any one of the other operational modes directly.

In some embodiments, an operator can designate an operational mode 4008 for each of the one or more arms of the robotic system. For example and without limitation, a first robotic arm holding an endoscope can be in the Passive Co-Manipulation Assistant mode 4010, a second robotic arm holding a surgical instrument can be in the Robotic Assistant mode 4014, and/or a third robotic arm can be in the Passive Assistant mode 4012. The operational mode 4008 of any one of the arms of the robotic system can be changed independent of the operational mode of the other arms 102 of the robotic system.

For example and without limitation, in the Passive Co-Manipulation Assistant mode 4010, the robotic system can be configured such that one or more arms of the robotic system can be simultaneously and freely moved by the user and moved based on algorithmic computations, such that the field of view of the endoscope can be changed by the user while the system simultaneously compensates for the effects of mass (i.e., inertia) and/or gravity during the movement - e.g., such that a mass or weight of the arm or arms of the robotic system is not detectable by an operator or is significantly attenuated. In some embodiments, the Passive Co-Manipulation Assistant mode 4010 can be used when an operator is user is actively manipulating an arm 102 of the robotic system. In a second example and without limitation, in the Passive Co-Manipulation Assistant mode 4010, the robotic system configuration can allow the user to freely move the retractor to a new position while simultaneously compensating for the mass or weight of the arm or arms of the robotic system and/or the surgical instrument (e.g., without limitation, a retractor) and/or an anatomical feature attached to the surgical instrument.

For example and without limitation, in the Passive Assistant mode 4012, the robotic system can be configured such that the arm or arms of the robotic system are static (i.e., not moving). The arm or arms that are in the Passive Assistant mode 4012 are essentially frozen, and no force is applied to the arm or arms externally by the operator. The robotic system can compensate for the force of gravity on the arm or arms that are in the Passive Assistant mode 4012. In some embodiments, when the operator exerts a force on an arm that is in the Passive Assistant mode 4012, the system can automatically change the operational mode 4008 controlling the arm to another operational mode 4008. For example and without limitation, the robotic system can change the operational mode 4008 controlling the arm from the Passive Assistant mode 4012 to the Passive Co-Manipulation Assistant mode 4010.

In some embodiments, the operational mode 4008 controlling the arm can be changed by an operator of the system using an input device or feature. For example and without limitation, the operational mode 4008 controlling the arm can be changed by an operator of the system 100 using a button on the arm (for example, button 149) or any other button, dial, or switch located on the arm or on the base 138 or otherwise, a foot pedal or foot switch, an input on a touchscreen, using gestures or force signatures (described in greater detail below), and/or any combination of the foregoing.

In the Robotic Assistant mode 4014, for example and without limitation, the robotic system can be configured such that the arms of the robotic system will move to reposition a surgical instrument, for example an endoscopic camera, based on algorithmic computations in order to change the field of view of the camera. In a second example and without limitation, in the Robot Assistant mode 4014, the robotic system can be configured such that the arms of the robotic system will move to reposition the surgical instrument, for example a laparoscopic retractor, based on algorithmic computations in order to increase the retraction force being applied to an anatomical feature,

For example and without limitation, in the Haptic mode 4016, the robotic system 100 can be configured such that the arm or arms of the robotic system 100 can be freely moved by the user to manipulate an instrument, which can be a surgical instrument, while the one or more motors of the arm simultaneously apply additional forces to the arm to reduce the effects of gravity, eliminate tremor of the instrument tip, avoidance of critical structures or to apply force feedback. A quick connector of the surgical instrument may automatically switch between robotic system modes. The sensors, motors, etc. of the robotic system may be active in all modes, but may act very differently (e.g., including acting as if inactive).

For some embodiments of the robotic system 100, a distinction between the Passive Assistant mode 4012 and the Haptic mode 4016 can be based on the instrument that is currently coupled with to the robotic system. For example, when the robotic system is in the "Passive Assistant" mode, the instrument can be a camera, a retractor, or a similar optical or surgical instrument, or, in some embodiments, any other device or component that a human assistant would typically hold. In some embodiments, when in the Haptic mode 4016, the instrument can be a surgical instrument such as a needle drive, scalpel, bipolar grasper, or similar surgical instrument. In some embodiments, the surgical instrument can be something that the primary human surgeon would typically hold in a non-robotic surgical operation. In the Haptic mode 4016, the system can provide gravity compensation to the arm and can provide one or more forces to the arm to prevent or inhibit particular movements in the arm (e.g., create one or more haptic barriers, which can be used to guide or direct the instrument toward a particular location, such as a trocar, or prevent or inhibit the instrument from moving into a particular area, such as an area occupied by an organ), etc.

In some embodiments, the robotic system does not replace the surgeon - instead, the robotic system augments the surgeon's capabilities through features like gravity compensation, tremor removal, haptic barriers, force feedback, etc.

With reference to Figure 15, in some embodiments of the mode of operation 4000, an operator can at step 4002 connect a surgical device or instruments to an arm, then at step 4004 select one of the four modes of operation 4008, which can be any of the following modes: Passive Co-Manipulation Assistant mode 4010, Passive Assistant mode 4012, Robot Assistant mode 4014, or Haptic mode 4016. The operator can also, at this step, change from any one of the four modes of operation 4008 any one of the other four modes of operation 4008. After one of the four modes of operation 4008 have been selected, the operator at step 4020 can use the robotic system. After the use of the robotic system at step 4020, the operator can at step 4020 remove the instrument or device from the arm. The operator can then terminate the procedure or perform any one of the steps 4002, 4004, 4020, and/or 4022 in any desired sequence.

In any embodiments disclosed herein, the robotic system can have less than four modes of operation 4008 or more than four modes of operation 4008. For example and without limitation, in some embodiments, the robotic system 100 can be configured to operate in one, two, or three of modes of operation 4008 described herein, and/or can be configured to operate in other modes of operation in addition to or in the alternative to any of the modes of operation 4008 disclosed herein. This can include a mode of operation in which the robotic arm is held completely stationary or fixed by the one or more motors and resists movement from forces imparted externally on the arm such as by an operator. This can prevent the arm and/or the tool attached to the arm from any or any significant movement if inadvertently bumped by an operator. In such cases, in this state, if the arm is inadvertently bumped or moved, the arm can be configured to inhibit any movement and can reposition itself in the precise position of the arm prior to the inadvertent force so that the position of the arm and/or the instrument is maintained despite inadvertent movement of the arm. In some embodiments, imparting a force on an arm of the robotic system when the system is in the passive assistant mode can cause the surgical robot to change the mode of operation to another mode of operation, including without limitation the co-manipulation assistant 4010 mode of operation.

In any embodiments disclosed herein, the robotic system can be configured to automatically change from one of the modes of operation 4008 to another of the modes of operation 4008 - e.g., without an additional input or communication from the operator. For example and without limitation, in some embodiments, the robotic system can be configured to change to a particular mode of operation 4008 based only the action of coupling a different instrument to the coupler. As described, any of the embodiments of the robotic system disclosed herein can be configured such that the robotic system can automatically identify the instrument that is coupled with the coupler and/or the robotic arm using an RFID transmitter chip and reader or receiver, an NFC device, a barcode and scanner or other optical device, a magnet based communication system, a Bluetooth transmitter, using the weight of the instrument and a lookup table, and/or any other features or mechanisms described herein or suitable for identification of the surgical instrument. Once the instrument is identified, the mode of operation 4008 that the robotic system transitions to (or remains in, if the robotic system is already in such mode of operation 4008) can be predefined and linked to the particular instrument that is coupled with the robotic arm of the system.

For example, if an operator attaches an endoscope to the robotic arm, the robotic system can be configured to change the mode of operation to the passive assistant mode 4012 or, if the robotic system is already in the passive assistant mode 4012, the robotic system can be configured to remain in the passive assistant mode 4012 if an endoscope is attached to the robotic arm. This configuration may be beneficial if, for example and without limitation, an endoscope is positioned at least partially within the body of a patient, but where the operator desires to change an orientation of the robotic arm that the endoscope is coupled with to avoid a collision or other interference, or to change the robotic arm that the endoscope is attached to. An operator could hold the instrument in the operator's hand, decouple the instrument from the robotic arm, rearrange the robotic arm or change the robotic arm to another robotic arm, and recouple the instrument. In the passive assistant mode 4012, the instrument can be held in the position that the instrument is in when coupled with the robotic arm.

In some embodiments, if an operator attaches an endoscope to the robotic arm but the endoscope is positioned outside of the body, the robotic system can be configured to change the mode of operation to the haptic mode 4016 or, if the robotic system is already in the haptic mode 4016, the robotic system can be configured to remain in the haptic mode 4016 if an endoscope that is positioned outside of the body is attached to the robotic arm, so that, for example and without limitation, the robotic system can guide the endoscope to the appropriate location.

As another example, in some embodiments, if an operator attaches a surgical instrument to the robotic arm, the robotic system can be configured to change the mode of operation to the co-manipulation assistant mode 4010 or, if the robotic system is already in the co-manipulation assistant mode 4010, the robotic system can be configured to remain in the co-manipulation assistant mode 4010 if a surgical tool is attached to the robotic arm.

In some embodiments, if an operator attaches an instrument to the robotic arm that the robotic system that an operator desires the robotic system to completely control, for example and without limitation, an irrigation device, the robotic system can be configured to change the mode of operation to the robot assistant mode 4014 or, if the robotic system is already in the robot assistant mode 4014, the robotic system can be configured to remain in the robot assistant mode 4014 in such circumstance. The robotic system can be configured to automatically robotically move the particular instrument to the desired position within the body.

In any embodiments disclosed herein, the robotic system can be configured to automatically change to the passive assistant 4012 mode of operation when an operator removes force from the robotic arm or instrument (e.g., when the operator removes the operators hands from the instrument or arm or stops moving the instrument or arm). Thereafter, the robotic system can be configured to maintain the robotic arm and/or surgical instrument in a fixed position.

### Indicators

In any embodiments disclosed herein, the system can have one or more lights used to identify the particular mode of operation associated with an arm of the system. The lights, which can be LED lights, can be located on an arm, on a console or interface for an operator, and/or on any other surface or component of the system. For example, each mode of operation 4008 can be associated with a uniquely colored light, such as red, yellow, blue, green, white, orange, etc. The lights can be configured to indicate a transition from a first mode of operation to a second mode of operation. For example and without limitation, if the system has a single indicator light configured to provide a plurality of different colors (each color indicating a different mode of operation), the light can be configured to transition from a first color (e.g., red) corresponding to the first mode of operation to the second color (e.g., yellow) corresponding to the second mode of operation by showing a transitionary color (e.g., orange) and/or by flashing.

### Gestures and Force Signatures

In any embodiments disclosed herein, an operator can also use predefined gestures to change the operational mode 4008 of the robotic system (any embodiment of the robotic system disclosed herein) and/or to indicate that the operator wishes to transition from one to another operational mode 4008. For example and without limitation, if an operator wishes to transition an arm of the robotic system to the Passive Co-Manipulation Assistant mode 4010, the operator can move an end portion of the respective arm in an up-and-down oscillating motion or movement pattern, if an operator wishes to transition an arm to the Passive Assistant mode 4012, the operator can move an end portion of the respective arm in a laterally oscillating motion (i.e., a side to side movement pattern), if an operator wishes to transition an arm to the Robot Assistant mode 4014, the operator can move an end portion of the respective arm in a circular motion, and/or if an operator wishes to transition an arm to the Haptic mode 4016, the operator can move an end portion of the respective arm in a triangular motion (i.e., a side to side movement pattern). Other motions or movement patterns can be used, such as an axial back and forth motion, by exerting a thumping or tapping force on the surgical instrument, the coupler, or an end portion of an arm, a sinusoidal motion or movement pattern, or any other desired movement pattern. In some embodiments, the motion or movement pattern can be any user induced repeatable motion having a pattern or signature (i.e., movement pattern) that can be detected by the processor of the robotic system. In some embodiments, the motion or movement pattern can be one that may not normally occur during a typical procedure. In any embodiments herein, a predefined gesture can be used to lock a particular arm 12 in a current or desired position, to unlock an arm, or otherwise. In any embodiments disclosed herein, the gestures can be determined from one or more sensors on the arm 102, encoder readings, motor current readings, and/or any combination of the foregoing.

In other embodiments, an operator can also impart a particular force on the arm to change the operational mode 4008 of the robotic system and/or to indicate that the operator wishes to transition from one to another operational mode 4008. For example and without limitation, an operator may exert a particular force on or near an end portion of the arm to indicate that the operator wishes to change the operation mode 4008 of the particular arm. Sensors or motor current readings can be used to detect the force and to determine if the force matches a predefined force signature associated with an operational change. If there is a match, then the system can change the operational mode 4008 of the arm to the particular operational mode 4008 that matches the force signature. In any embodiments disclosed herein, the system can have one or more force and/or torque sensors at any of the articulation joints (which can include, for example, articulation joint 136) and/or one or more force sensors in a distal end portion of an arm to detect and measure a force applied to an arm of the system, which measured force can be compared with one or more predefined force signatures stored in the system to determine if the applied force matches one of the predefined force signatures. If there is a match, the system can change the operational mode 4008 of the arm that the force was applied to with the operational mode 4008 corresponding to the force signature.

Some embodiments include one arm, two arms 102, three arms 102, four arms 102, or more. The robotic system 100 may comprise two bases each with one or more arms 102. For example, a robotic system 100 may comprise a first base comprising two arms 102 and a second base comprising two arms 102 for a total of four arms 102. Because a centralized system receives data from and provides force to each of the four arms 102, it may be considered a single four-armed robotic system 100 even though the arms 102 are connected to different bases. Various arms 102 may be configured differently, for example as described herein.

While certain embodiments have been described, these embodiments have been presented by way of example only, and are not intended to limit the scope of the disclosure. Indeed, the methods and systems described herein may be embodied in a variety of other forms. Various omissions, substitutions and changes in the systems and methods described herein may be made without departing from the spirit of the disclosure. The accompanying claims and their equivalents are intended to cover such forms or modifications as would fall within the scope and spirit of the disclosure.

Features, materials, characteristics, or groups described in conjunction with a particular aspect, embodiment, or example are to be understood to be applicable to any other aspect, embodiment or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

Certain features that are described in this disclosure in the context of separate implementations can also be implemented in combination in a single implementation. Conversely, various features that are described in the context of a single implementation can also be implemented in multiple implementations separately or in any suitable subcombination. Although features may be described above as acting in certain combinations, one or more features from a claimed combination can, in some cases, be excised from the combination, and the combination may be claimed as a subcombination or variation of a subcombination.

While operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. The operations may be rearranged or reordered in other implementations. Those skilled in the art will appreciate that in some embodiments, the actual steps taken in the processes illustrated and/or disclosed may differ from those shown in the figures. Depending on the embodiment, certain of the steps described above may be removed, others may be added. The features and attributes of the specific embodiments disclosed above may be combined in different ways to form additional embodiments, all of which fall within the scope of the present disclosure. Also, the separation of various system components in the implementations described above should not be understood as requiring such separation in all implementations, and it should be understood that the described components and systems can generally be integrated together in a single product or packaged into multiple products.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" represent a value, amount, or characteristic close to the stated value, amount, or characteristic that still performs a desired function or achieves a desired result. For example, the terms "approximately," "about," "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree. The ranges disclosed herein also encompass any and all overlap, sub-ranges, and combinations thereof, and any specific values within those ranges. Language such as "up to," "at least," "greater than," "less than," "between," and the like includes the number recited. Numbers and values used herein preceded by a term such as "about" or "approximately" include the recited numbers. For example, "approximately 7 mm" includes "7 mm" and numbers and ranges preceded by a term such as "about" or "approximately" should be interpreted as disclosing numbers and ranges with or without such a term in front of the number or value such that this application supports claiming the numbers, values and ranges disclosed in the specification and/or claims with or without the term such as "about" or "approximately" before such numbers, values or ranges such, for example, that "approximately two times to approximately five times" also includes the disclosure of the range of "two times to five times." The methods disclosed herein may include certain actions taken by a practitioner; the methods can also include any third-party instruction of those actions, either expressly or by implication. For example, actions such as "performing a surgical operation" include "instructing performing a surgical operation." The scope of the present disclosure is not intended to be limited by the specific disclosures of preferred embodiments in this section or elsewhere in this specification, and may be defined by claims as presented in this section or elsewhere in this specification or as presented in the future. The language of the claims is to be interpreted broadly based on the language employed in the claims and not limited to the examples described in the present specification or during the prosecution of the application, which examples are to be construed as non-exclusive.

## Claims

1. A device for coupling an instrument to an arm of a surgical robot, comprising:
a body configured to selectively couple with an instrument for use in a surgical operation;
an interface configured to selectively couple with the body and configured to be coupled with an end portion of a robotic arm;
wherein:
the device is configured to permit the instrument to rotate about a longitudinal axis of the instrument relative to the device;
the device is configured to inhibit longitudinal movement of the instrument relative to the device.

2. The device of claim 1, wherein the body is configured to clamp around a portion of an outside surface of the instrument.

3. The device of any one of the previous claims, wherein the body comprises a first portion coupled with a second portion with a hinge, wherein the first portion can rotate about the hinge relative to the second portion so as to selectively clamp the instrument in a recess formed in the body.

4. The device any one of the previous claims, wherein the body is configured to clamp around a portion of an outside surface of the instrument and to prevent a rotational movement of the instrument relative to the body under normal operating conditions.

5. The device of any one of the previous claims, wherein the interface comprises a recess configured to removably receive the body therein.

6. The device of claim 5, wherein the recess of the interface is configured to inhibit longitudinal movement of the body relative to the interface and to permit rotational movement of the body relative to the interface.

7. The device of any one of the previous claims, configured to move between a first state in which the instrument is removable from the device and a second state in which the instrument is nonremovable from the device.

8. The device of any one of the previous claims, wherein the body has one or more projections extending away from a surface of the body and the interface can have one or more depressions configured to receive the one or more projections to align the body with the interface.

9. The device of any one of the previous claims, wherein the instrument is a laparoscopic surgical instrument or an endoscope.

10. A co-manipulation robotic surgical device for manipulating an instrument, comprising:
a base portion;
a first arm coupled with the base portion;
a motor coupled with the first arm and configured to rotate the first arm relative to the base portion;
an instrument coupled with an end portion of the first arm; and
a controller configured to control the first arm according to at least two of the following operational modes:
passive assistant mode;
co-manipulation assistant mode;
robotic assistant mode; and
haptic mode;
wherein:
in the passive assistant mode, the first arm is static;
in the co-manipulation assistant mode, the first arm is freely movable by an operator while the motor at least partially simultaneously moves the first arm to improve a position and/or orientation of the instrument coupled with the end portion of the first arm and/or to compensate at least for a force of gravity on the first arm and the instrument that is coupled with the end portion of the first arm;
in the robotic assistant mode, the motor moves the first arm to reposition the instrument coupled with the end portion of the first arm; and
in the haptic mode, the first arm is movable by an operator while the motor compensates at least for a force of gravity on the first arm and/or the instrument that is coupled with the end portion of the first arm and at least guides the instrument along a predefined trajectory, prevents unwanted movements of the first arm and/or the instrument coupled with the end portion of the first arm, prevents a movement of the first arm outside of a particular space, and/or prevents a movement of the first arm into a particular space.

11. The co-manipulation robotic surgical device of claim 10, wherein the controller is switchable between any one of at least three of the operational modes.

12. The co-manipulation robotic surgical device of claim 10, wherein the controller is switchable between any one of the four operational modes.

13. The co-manipulation robotic surgical device of claim 12, wherein the co-manipulation robotic surgical device is configured to automatically identify the particular instrument that is coupled with the end portion of the first arm using an RFID transmitter chip, a barcode, a near field communication device, a Bluetooth transmitter, and/or a weight of the instrument that is coupled with the end portion of the first arm.

14. The co-manipulation robotic surgical device of any one of claims 10-13, wherein the co-manipulation robotic surgical device is configured to automatically change to a predetermined one of the operational modes when a particular instrument is coupled with the end portion of the first arm without any additional input from an operator.

15. The co-manipulation robotic surgical device of any one of claims 10-14, wherein the co-manipulation robotic surgical device is configured to change to the passive assistant mode when a particular instrument is coupled with the end portion of the first arm without any additional input from an operator.
